# EUROPEAN PATENT APPLICATION

(11) **EP 3 957 326 A1**
(43) Date of publication of application: **23.02.2022**
(21) Application number: 20791286.6
(22) Date of filing: 16.04.2020
(51) Int. Cl.: A61K 39/395, C07D 471/04, C12Q 1/6886, A61P 35/00

(54) **USE OF ANTI-PD-1 ANTIBODY IN PREPARATION OF MEDICAMENT FOR TREATING SOLID TUMORS**

(30) Priority: 17.04.2019 CN 201910310345
(71) Applicant: Shanghai Junshi Biosciences Co., Ltd., Pilot Free Trade Zone Shanghai 201210 (CN)
(72) Inventor: FENG, Hui, Shanghai 201210 (CN); WU, Hai, Shanghai 201210 (CN); YAO, Sheng, Shanghai 201210 (CN)
(74) Representative: V.O.
(86) International application number: PCT/CN2020/085046
(87) International publication number: WO 2020/211804

(57) **Abstract**

The present invention relates to use of an anti-PD-1 antibody in the treatment of a tumor. The present invention also relates to use of a reagent for detecting a gene amplification in the chromosome 11q13 region in a test kit for predicting the therapeutic effect of the anti-PD-1 antibody and/or the antigen-binding fragment thereof on a tumor patient.

## Description

### TECHNICAL FIELD

The present invention relates to use of an anti-PD-1 antibody in the treatment of a tumor. In particular, the present invention relates to use of an anti-PD-1 antibody in the treatment of a solid tumor, preferably esophageal cancer, more preferably esophageal squamous cell carcinoma (ESCC); use of the anti-PD-1 antibody in the preparation of a medicament for treating a solid tumor, preferably esophageal cancer, more preferably esophageal squamous cell carcinoma (ESCC); a method for predicting the therapeutic effect of the anti-PD-1 antibody on a solid tumor, preferably esophageal cancer, more preferably esophageal squamous cell carcinoma (ESCC) using biomarkers; and use of a combination therapy or composition comprising the anti-PD-1 antibody in the treatment of a solid tumor.

### BACKGROUND

Immune escape is one of the characteristics of cancer. Ahmadzadeh, M. et al, Blood, 114: 1537-44 disclosed that tumor-specific T lymphocytes are often present in the tumor microenvironment, draining lymph nodes and peripheral blood, but are generally unable to control tumor progression due to the network of immunosuppressive mechanisms present in the tumor microenvironment. CD8⁺ tumor infiltrating T lymphocytes (TILs) typically express activation-induced inhibitory receptors, including CTLA-4 and PD-1, while tumor cells often express immunosuppressive ligands, including PD-1 ligand 1 (PD-L1, also called B7-H1 or CD274), which inhibits activation and effector functions of T cells. In the inhibitory mechanism, PD-1 and its ligands have become an important pathway for tumor cells to suppress activated T cells in the tumor microenvironment.

Programmed death receptor 1 (PD-1) plays an important role in immune regulation and maintenance of peripheral tolerance. PD-1 is expressed primarily in activated T and B cells and functions to suppress lymphocyte activation, which is a normal peripheral tissue tolerance mechanism of the immune system that prevents over-reactive immunity. However, the activated T cells infiltrated in the tumor microenvironment highly express PD-1 molecules, and inflammatory factors secreted by the activated leukocytes can induce the tumor cells to highly express ligands PD-L1 and PD-L2 of PD-1, resulting in the continuous activation of the PD-1 pathway of the activated T cells in the tumor microenvironment, and the suppression of T cells function to kill tumor cells. Therapeutic PD-1 antibodies can block this pathway, partially restore the function of T cells, and enable the activated T cells to continuously kill tumor cells.

Blocking the PD-1/PD-L1 pathway has proven to be an effective way to induce a durable anti-tumor response in various cancer indications over the last decade. Monoclonal antibodies (mAbs) blocking the PD/PD-L1 pathway can enhance activation and effector functions of tumor specific T cells, reduce tumor burden, and improve survival rate.

Esophageal cancer (EC) is one of the most common malignancies in humans, the morbidity and mortality of which has continued to rise over the past few decades, with 400,000 deaths worldwide per year now. Esophageal squamous cell carcinoma (ESCC) is the most common histological subtype of esophageal cancer in developing countries, and is the dominant histological subtype of esophageal cancer in South American and East Asian populations. The need for the treatment of this tumor remains unmet worldwide. In China, EC is the third most common cancer and the fourth leading cause of cancer-related death. ESCC accounts for more than 90% of the total esophageal cancers in China and is commonly treated with chemotherapy and radiotherapy. The most commonly used chemotherapeutic agents for metastatic ESCC are cisplatin, 5-fluorouracil and taxanes. The 5-year survival rate of ESCC patients is low to 15%-20%. Currently, there is no standard therapy for chemotherapy-refractory EC patients. Recently, Clin Cancer Res 2018; 24(6) 1296-304 disclosed that an anti-PD-1 antibody SHR-1210 has controllable safety and effective anti-tumor activity for treating chemotherapy-refractory ESCC patients in China, and found that PD-L1 positive tumors have a higher objective response rate than PD-L1 negative tumors, and tumor mutation burden (TMB) and the number of potential mutation-related neoantigens are associated with better therapeutic response. In addition, J Clin Oncol 2019; 37, 2019 (suppl 4; abstr 2) disclosed that advanced ESCC patients with disease progression after first-line treatment have significant overall survival using a pamtuzumab treatment compared to chemotherapy in the population with a PD-L1 combined positive score (CPS) ≥ 10. Therefore, although previous studies have demonstrated the efficacy of PD-1 targeted therapy in patients of metastatic ESCC subpopulations, effective predictive biomarkers for anti-PD-1 antibody immunotherapy are not yet clear.

### SUMMARY

In a first aspect of the present invention, provided is use of an anti-PD-1 antibody or an antigen-binding fragment thereof administered alone or in combination with an additional anti-cancer agent, in the preparation of a medicament for treating cancer.

In one or more embodiments, the additional anti-cancer agent described herein is a small molecule targeted anti-cancer agent. In one embodiment, the additional anti-cancer agent described herein is selected from a CDK4/6 inhibitor and an FGF/FGFR inhibitor.

In one or more embodiments, the medicament comprises the anti-PD-1 antibody or the antigen-binding fragment thereof and the CDK4/6 inhibitor. In one or more embodiments, the medicament comprises the anti-PD-1 antibody or the antigen-binding fragment thereof and the FGF/FGFR inhibitor.

In one or more embodiments, the present invention provides use of an anti-PD-1 antibody or an antigen-binding fragment thereof in the preparation of a medicament for treating a cancer patient in combination with a CDK4/6 inhibitor or an FGF/FGFR inhibitor.

In one or more embodiments, the present invention provides use of an anti-PD-1 antibody or an antigen-binding fragment thereof in combination with a CDK4/6 inhibitor or an FGF/FGFR inhibitor, in the preparation of a medicament for treating a cancer patient.

In one or more embodiments, the cancer described herein is a solid tumor.

In one or more embodiments, the cancer includes, but is not limited to, gastric cancer, esophageal cancer, nasopharyngeal cancer, head and neck squamous cell carcinoma, breast cancer, bladder cancer, and colon cancer.

In one or more embodiments, the cancer is preferably esophageal cancer. In one or more preferred embodiments, the cancer is esophageal squamous cell carcinoma. In one or more preferred embodiments, the cancer is advanced ESCC. In one or more preferred embodiments, the cancer is chemotherapy-refractory ESCC. In other embodiments, the esophageal squamous cell carcinoma is advanced, metastatic and/or refractory esophageal squamous cell carcinoma.

In one or more embodiments, the solid tumor does not have a gene amplification of the chromosome 11q13 region.

In one or more embodiments, the present invention provides use of an anti-PD-1 antibody (preferably toripalimab) in combination with a CDK4/6 inhibitor (preferably ribociclib or palbociclib, more preferably palbociclib) in the preparation of a medicament for treating esophageal cancer or colon cancer; in some embodiments, the present invention provides use of an anti-PD-1 antibody (preferably toripalimab) in the preparation of a medicament for treating esophageal cancer.

In certain embodiments, the individual has received a prior treatment. In certain embodiments, the prior treatment includes, but is not limited to, chemotherapy or radiotherapy. In certain embodiments, the individual has received a systemic treatment. In one or more preferred embodiments, the prior treatment or the systemic treatment is performed at least twice. In certain embodiments, the individual does not have autoimmune disease or history of autoimmune disease. In certain embodiments, the individual does not have any concomitant disease requiring long-term immunosuppressive drug treatment. In certain embodiments, the individual has not received a prior treatment with any immune checkpoint blocker.

In some embodiments, the anti-PD-1 antibody or the antigen-binding fragment thereof is administered alone or in combination with an additional anti-cancer agent to induce a durable clinical response in the individual.

In one or more embodiments, in the treatment with the anti-PD-1 antibody or the antigen-binding fragment thereof administered alone or in combination with an additional anti-cancer agent, the anti-PD-1 antibody or the antigen-binding fragment thereof is intravenously infused at a therapeutically effective dose of about 0.1 mg/kg body weight to about 10.0 mg/kg body weight about once every 2 weeks. In some embodiments, the anti-PD-1 antibody or the antigen-binding fragment thereof is administered at a dose of about 1 mg/kg body weight, 3 mg/kg body weight or 10 mg/kg body weight, or of a fixed dose of 240 mg/kg once every 2 weeks.

In one or more embodiments, the anti-PD-1 antibody or the antigen-binding fragment thereof is administered at a single dose of about 0.1 mg/kg body weight to about 10.0 mg/kg body weight, e.g., about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight, or selected from a fixed dose of about 120 mg to about 480 mg, e.g., a fixed dose of 120 mg, 240 mg, 360 mg or 480 mg.

In one or more embodiments, the anti-PD-1 antibody or the antigen-binding fragment thereof is administered at a frequency of about once every week, once every two weeks, once every three weeks, once every four weeks or once a month, preferably once every two weeks.

In one or more embodiments, the anti-PD-1 antibody or the antigen-binding fragment thereof is administered at a single dose of 1 mg/kg body weight, 3 mg/kg body weight or 10 mg/kg body weight, or of a fixed dose of 240 mg once every two weeks.

In one or more embodiments, the anti-PD-1 antibody or the antigen-binding fragment thereof is administered parentally, e.g., by intravenous infusion, in a liquid dosage form, e.g., an injection.

In one or more embodiments, the anti-PD-1 antibody or the antigen-binding fragment thereof can be administered for a period of one week, two weeks, three weeks, one month, two months, three months, four months, five months, half a year or longer, optionally, the duration of each administration period can be the same or different, and the interval between each administration period can be the same or different.

In a second aspect of the present invention, provided is a kit for treating an individual with cancer, comprising: (a) a monoclonal antibody or an antigen-binding fragment thereof that specifically binds to and inhibits PD-1, and optionally an additional anti-cancer agent other than the anti-PD-1 antibody or the antigen-binding fragment thereof; and (b) instructions for administering the anti-PD-1 antibody or the antigen-binding fragment thereof that specifically binds to and inhibits PD-1 alone, or in combination with the additional anti-cancer agent, to treat cancer in the individual. The cancer is a solid tumor. As a preferred embodiment, the cancer includes, but is not limited to, gastric cancer, esophageal cancer, nasopharyngeal cancer, head and neck squamous cell carcinoma, breast cancer, bladder cancer, and colon cancer. As a preferred embodiment, the cancer is preferably esophageal cancer. As a preferred embodiment, the cancer is esophageal squamous cell carcinoma. As a further preferred embodiment, the cancer is ESCC. As a preferred embodiment, the solid tumor does not have a gene amplification of the chromosome 11q13 region.

In one or more embodiments, the additional anti-cancer agent described herein is a small molecule targeted anti-cancer agent. In one embodiment, the additional anti-cancer agent described herein is selected from one or more of a CDK4/6 inhibitor and an FGF/FGFR inhibitor.

In one or more embodiments, the kit comprises the anti-PD-1 antibody or the antigen-binding fragment thereof and the CDK4/6 inhibitor. In one or more embodiments, the kit comprises the anti-PD-1 antibody or the antigen-binding fragment thereof and the FGF/FGFR inhibitor.

In a third aspect of the present invention, provided is use of a CDK4/6 inhibitor or an FGF/FGFR inhibitor in the preparation of a medicament for treating cancer.

In a fourth aspect of the present invention, provided is a method for treating cancer, comprising sequencing an individual prior to treatment; wherein an individual not having an amplification of the chromosome 11q13 region is administered with an anti-PD-1 antibody or an antigen-binding fragment thereof alone, optionally in combination with one of or a combination of more of a CDK4/6 inhibitor and/or an FGF/FGFR inhibitor; an individual having an amplification of the chromosome 11q13 region is administered with one of or a combination of more of a CDK4/6 inhibitor and/or an FGF/FGFR inhibitor alone, optionally in combination with an anti-PD-1 antibody or an antigen-binding fragment thereof.

In the uses, methods, medicaments and kits described herein, the individual is a human.

In one or more preferred embodiments, the individual described herein is a human and the cancer is a solid tumor. In one or more preferred embodiments, the individual described herein is a human and the cancer is selected from, but is not limited to, gastric cancer, esophageal cancer, nasopharyngeal cancer, head and neck squamous cell carcinoma, breast cancer, bladder cancer, and colon cancer. In a preferred embodiment, the individual described herein is a human and the cancer is esophageal cancer. In a preferred embodiment, the individual described herein is a human and the cancer is ESCC. In one or more embodiments, the individual described herein has esophageal cancer and has not received prior immunotherapy.

In a fifth aspect of the present invention, provided is a method for predicting the therapeutic effect of the anti-PD-1 antibody in a tumor patient, comprising determining whether the patient has a gene amplification of the chromosome 11q13 region, wherein the absence of the gene amplification of the chromosome 11q13 region indicates that the tumor patient is suitable for treatment with the anti-PD-1 antibody.

In the uses, methods, medicaments and kits described herein, the anti-PD-1 antibody is a monoclonal antibody or an antigen binding fragment thereof. In certain embodiments, the anti-PD-1 antibody specifically binds to PD-1 and blocks the binding of PD-L1 or PD-L2 to PD-1. In certain embodiments, the anti-PD-1 antibody specifically binds to PD-L1 or/and PD-L2 and blocks the binding of PD-L1 and/or PD-L2 to PD-1.

In one or more embodiments, the anti-PD-1 antibody comprises complementarity determining regions (CDRs), wherein light chain complementarity determining regions (LCDRs) comprise amino acid sequences set forth in SEQ ID NOs: 1, 2 and 3, and heavy chain complementarity determining regions (HCDRs) comprise amino acid sequences set forth in SEQ ID NOs: 4. 5 and 6.

In one or more embodiments, the anti-PD-1 antibody comprises a light chain variable region (VL) set forth in SEQ ID NO: 7, and a heavy chain variable region (VH) set forth in SEQ ID NO: 8.

In one or more embodiments, the anti-PD-1 antibody comprises a light chain and a heavy chain, wherein the light chain comprises an amino acid sequence set forth in SEQ ID NO: 9, and the heavy chain comprises an amino acid sequence set forth in SEQ ID NO: 10.

### BRIEF DESCRIPTION OF THE DRAWINGS

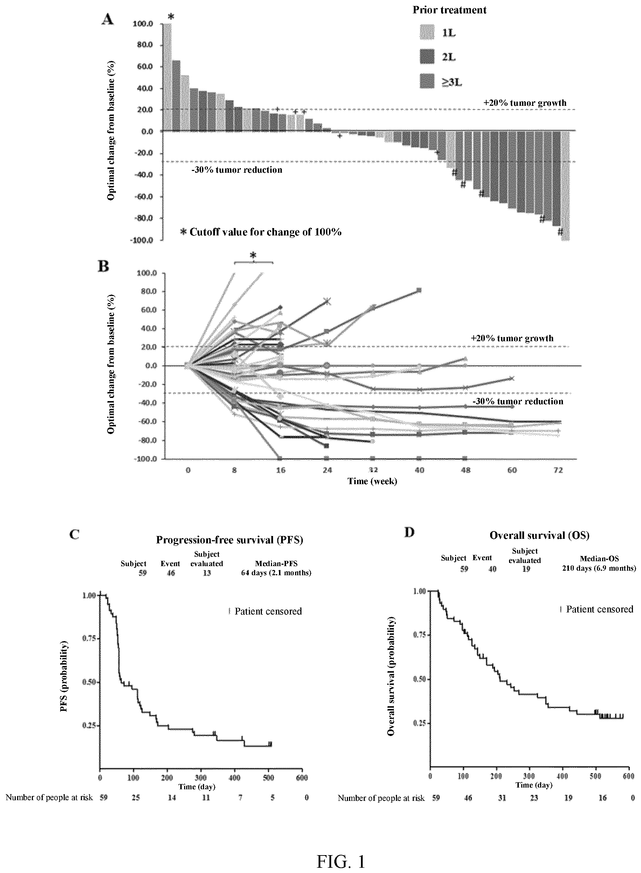

FIG. 1 shows: (A) Maximum change in tumor size compared to baseline as assessed by investigators according to RECIST v1.1 (n = 46, subjects have baseline and at least one post-treatment imaging evaluation). The length of the bar graph indicates the maximum decrease or minimum increase in the target lesion. The color of the bar graph reflects the results of prior systemic treatments. Blue: ≥ 3 L; orange: 2 L; green: 1 L. In 5 patients (marked #), the target lesion changes best from baseline with the tumor reduction greater than 30%, but the response cannot confirm the possible occurrence of new lesions. In 5 patients (marked +), the target lesion changes best from baseline with the tumor growth less than 20%, but features progressive disease (PD) due to the occurrence of new lesions or the progression of non-target lesions.
   (B) Changes in tumor burden of each individual over time assessed by investigators according to RECIST v1.1 (n = 46, subjects have baseline and at least one post-treatment X-ray film evaluation).* The cutoff value for change percentage is 100%.
   (C) Progression-free survival of all patients in the study.
   (D) Overall survival of all patients in the study. The percentage of surviving patients at the indicated time points is shown. The patient censored is marked " | " in the figure. The number of patients at risk at a given time point is shown below the X-axis.

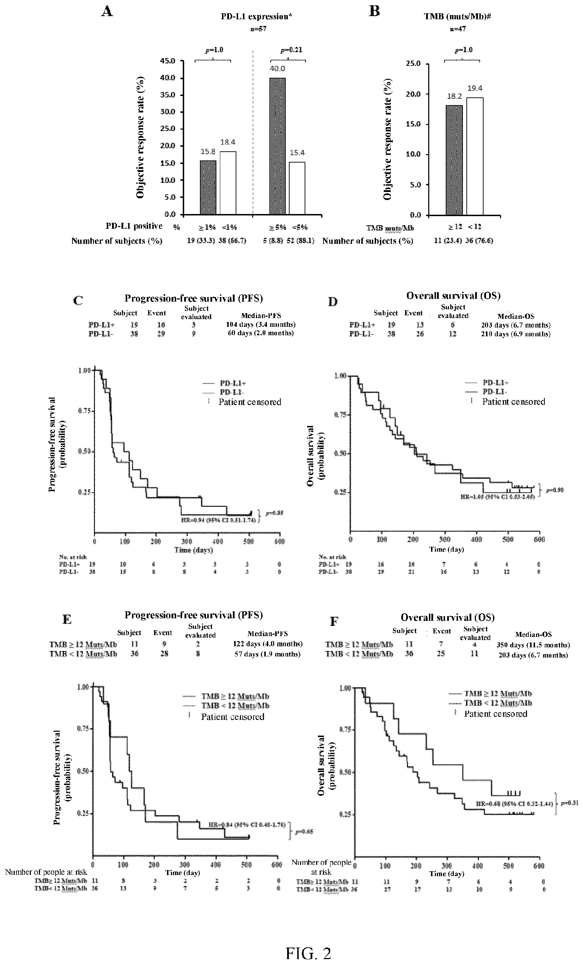

FIG. 2 shows clinical responses associated with tumor PD-L1 expression or tumor mutation burden (TMB).
   (A) PD-L1 positive status is defined as the presence of tumor cells or immune cells having the membrane staining intensity ≥ 1% by SP142 IHC staining.
   (B) TMB status determined by whole exome sequencing of tumor biopsies and paired PBMCs.
   (C) Comparison of PFS of PD-L1 positive patients and PD-L1 negative patients.
   (D) Comparison of OS of PD-L1 positive patients and PD-L1 negative patients.
   (E) Comparison of PFS of patients with high TMB and patients with low TMB.
   (F) Comparison of OS of patients with high TMB (≥ 12) and patients with low TMB (< 12). The percentage of surviving patients at the indicated time points is shown. The patient censored is marked " | " in the figure. The number of patients at risk at a given time point is shown below the X-axis.
FIG. 3 shows mutations in advanced EC patients.
   Genomic map of WES shows that missense mutations or truncations of TP53 (p53) (76%), RYR2 (22%), NOTCH1 (20%), LRP1B (17%) and TRIO (17%) are the most common 5 mutations in ESCC tumor biopsies.
FIG. 4 shows the amplification of 11q13 gene locus and RNA expression analysis of enrolled ESCC patients.
   (A) Genomic profiling performed by the next-generation sequencing of FFPE tumors and paired peripheral blood samples from 51 available patients. The amplification of 11q13 occurs in 24 patients. The top shows the chromosome 11q13 region and the encoding gene. Three algorithms are used to determine the amplification event. Each individual has an amplified gene. Mutations or deletions in the amplified gene are also marked.
   (B) Messenger RNA expression analysis of amplified 11q13. mRNA sequencing and expression profiling are performed on existing patients. mRNA expression level is associated with gene amplification in each individual.

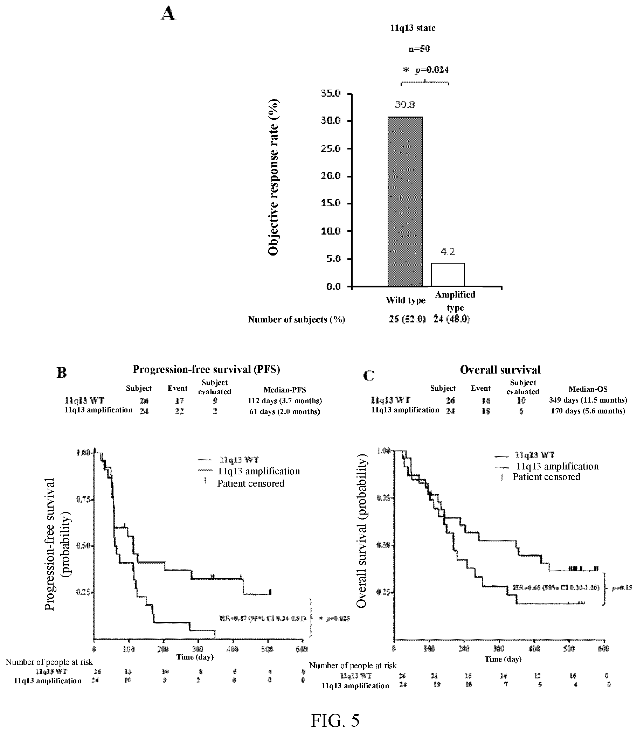

FIG. 5 shows the correlation of clinical response with gene amplification status of the chromosome 11q13 region.
   (A) Tumor 11q13 status determined by whole exome sequencing of tumor biopsies and paired PBMCs.
   (B) Progression-free survival of patients with 11q13 wild type vs. patients with 11q13 amplified type.
   (C) Overall survival of patients with 11q13 wild type vs. patients with 11q13 amplified type. The percentage of surviving patients at the indicated time points is shown. The patient censored is marked " | " in the figure. The number of patients at risk at a given time point is shown below the X-axis.

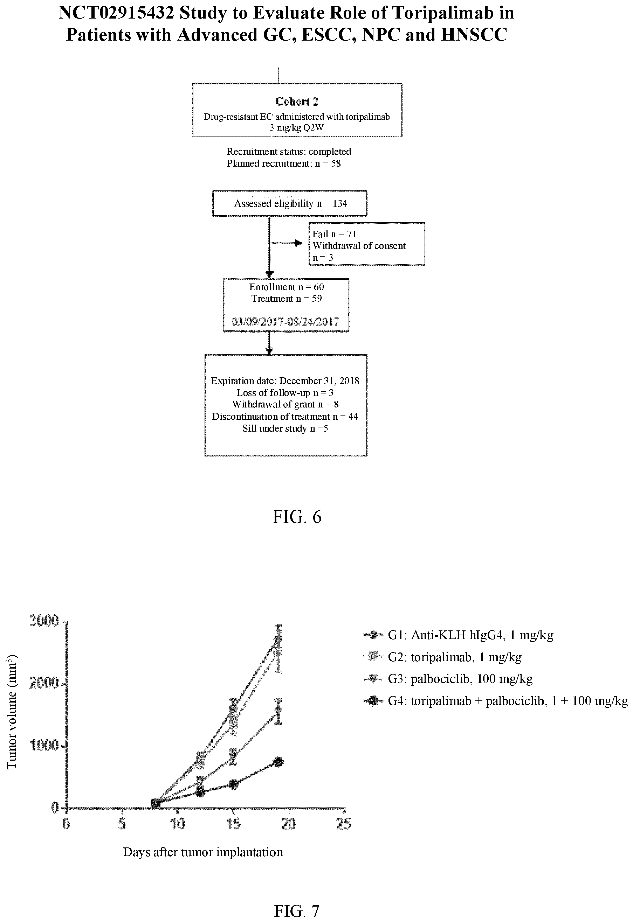

FIG. 6 shows the course of the NCT02915432 clinical trial evaluating the effect of toripalimab in patients with advanced GC, ESCC, NPC and HNSCC.
FIG. 7 shows the study results of the inhibitory effect of the anti-PD-1 antibody and a CDK4/6 inhibitor on tumor growth in mice.

### DETAILED DESCRIPTION

The present invention relates to a method for treating tumors. The method of the present invention comprises administering to a patient in need the anti-PD-1 antibody or the antigen-binding fragment thereof alone; or comprises administering to a patient in need the anti-PD-1 antibody or the antigen-binding fragment thereof in combination with an additional anti-cancer agent. The present invention also relates to a method for predicting the therapeutic effect of the anti-PD-1 antibody on a cancer patient, preferably an esophageal cancer patient using biomarkers.

### Terminology

In order to facilitate the understanding of the present invention, some technical and scientific terms are specifically defined below. Unless otherwise specifically defined herein, all technical and scientific terms used herein have the same meaning as commonly understood by those of ordinary skill in the art to which the present invention belongs.

"Administering", "giving" and "treating" refers to introducing a composition comprising a therapeutic agent into a subject using any one of a variety of methods or delivery systems known to those skilled in the art. Routes of administration of the anti-PD-1 antibody include intravenous, intramuscular, subcutaneous, intraperitoneal, spinal or other parenteral routes of administration, such as injection or infusion. "Parenteral administration" refers to modes of administration apart from enteral or local administration, typically by injection, including but not limited to, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, and *in vivo* electroporation.

An "adverse event" (AE) described herein is any adverse and often unintended or undesirable sign, symptom, or disease associated with the use of medical treatment. For example, an adverse event may be associated with the activation of the immune system or the expansion of immune system cells in response to treatment. The medical treatment may have one or more related AEs, and each AE may have the same or a different severity level.

The term "subject" includes any organism, preferably an animal, more preferably a mammal (such as rat, mouse, dog, cat and rabbit), and most preferably a human. The terms "subject", "patient" and "individual" are used interchangeably herein.

An "antibody" described herein refers to any form of antibody that achieves a desired biological or binding activity. Therefore, it is used in the broadest sense, but is not limited to, monoclonal antibodies, polyclonal antibodies, multispecific antibodies, humanized full-length human antibodies, chimeric antibodies and camelized single-domain antibodies, which specifically bind to an antigen and comprise at least two heavy (H) and two light (L) chains interconnected by disulfide bonds, or antigen-binding fragments thereof. Each heavy chain comprises a heavy chain variable region (VH) and a heavy chain constant region comprising three constant domains CHI, CH2 and CH3. Each light chain comprises a light chain variable region (VL) and a light chain constant region comprising one constant domain CL. The VH and VL regions can be further divided into hypervariable regions termed complementarity determining regions (CDRs), which are scattered over more conserved regions termed framework regions (FRs). Generally, both light and heavy chain variable domains comprise FR1, CDR1, FR2, CDR2, FR3, CDR3 and FR4 from N-terminus to C-terminus. Amino acids are typically assigned to each domain according to the following definitions: Sequences of Proteins of Immunological Interest, Kabat et al; National Institutes of Health, Bethesda, Md.; 5th edition; NIH publication No. 91-3242 (1991): Kabat (1978) Adv. Prot. Chem. 32:1-75; Kabat et al, (1977) J. Biol. Chem. 252:6609-6616; Chothia et al, (1987) J Mol. Biol. 196:901-917 or Chothia et al, (1989) Nature 341:878-883.

The carboxyl-terminal portion of the heavy chain can define a constant region primarily responsible for effector function. Human light chains are generally classified as κ and λ chains. Human heavy chains are generally classified as µ, δ, γ, α or ε chains, and isotypes of the antibody are defined as IgM, IgD, IgG, IgA and IgE, respectively. IgG subclass is well known to those skilled in the art and includes, but is not limited to, IgG1, IgG2, IgG3 and IgG4.

The term "antibody" includes: naturally occurring and non-naturally occurring Abs; monoclonal and polyclonal Abs; chimeric and humanized Abs; human or non-human Abs; fully synthetic Abs; and single chain Abs. Non-human Abs can be humanized by recombinant methods to reduce their immunogenicity in humans.

Unless otherwise specifically indicated, an "antibody fragment" or "antigen-binding fragment" described herein refers to an antigen-binding fragment of an antibody, i.e., an antibody fragment that retains the ability of a full-length antibody to specifically bind to an antigen, e.g., a fragment that retains one or more CDR regions. Examples of binding fragments of an antibody include, but are not limited to, Fab, Fab', F(ab')₂, and Fv fragments; a diabody; a linear antibody; a single-chain antibody molecule; and a nanoantibody and a multispecific antibody formed from fragments of the antibody.

A "chimeric antibody" refers to an antibody and a fragment thereof in which a portion of the heavy and/or light chain is identical with or homologous to corresponding sequences of an antibody derived from a particular species (e.g., human) or belonging to a particular antibody class or subclass, while the remainder of the chain is identical with or homologous to corresponding sequences of an antibody derived from another species (e.g., mouse) or belonging to another antibody class or subclass, so long as they exhibit the desired biological activity.

A "human antibody" refers to an antibody that comprises only human immunoglobulin sequences. A human antibody may contain a murine carbohydrate chain if it is produced in mice, mouse cells, or hybridomas derived from mouse cells. Similarly, "mouse antibody" or "rat antibody" refers to an antibody that comprises only mouse or rat immunoglobulin sequences, respectively.

A "humanized antibody" refers to an antibody form containing sequences from both non-human (e.g., murine) and human antibodies. Such antibodies contain minimal sequences derived from non-human immunoglobulins. Typically, a humanized antibody will comprise substantially all of at least one and typically two variable domains, in which all or substantially all of the hypervariable loops correspond to those of a non-human immunoglobulin and all or substantially all of the FR regions are those of a human immunoglobulin. The humanized antibody optionally also comprises at least one portion of an immunoglobulin constant region (Fc), typically a human immunoglobulin constant region.

The term "cancer" used herein refers to a wide variety of diseases characterized by the uncontrolled growth of abnormal cells in the body. Unregulated cell division, growth division and growth lead to the formation of malignancies that invade adjacent tissues and can also metastasize to distal parts of the body through the lymphatic system or the blood flow. Examples of cancer include, but are not limited to, carcinoma, lymphoma, leukemia, blastoma, and sarcoma. More specific examples of cancer include squamous cell cancer, myeloma, small-cell lung cancer, non-small cell lung cancer, glioma, Hodgkin lymphoma, non-Hodgkin lymphoma, acute myeloid leukemia, multiple myeloma, gastrointestinal cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, gastric cancer, bladder cancer, hepatoma, breast cancer, colon cancer, and head and neck cancer. Another embodiment of cancer includes esophageal cancer. Another specific embodiment of cancer includes ESCC. Another specific embodiment of cancer includes chemotherapy-refractory ESCC. Another specific embodiment of cancer includes advanced ESCC. In certain embodiments, the cancer described herein includes those characterized in that the sequenced individual has a gene amplification of the chromosome 11q13 region. The cancer described herein includes those characterized in that the sequenced individual does not have a gene amplification of the chromosome 11q13 region.

The term "esophageal cancer" or "EC" is one of the most common malignancies in humans. It is a common gastrointestinal tumor, and can be divided into squamous cell carcinoma, adenocarcinoma, and small cell carcinoma according to histological and pathological typing. Among them, "esophageal squamous cell carcinoma" or "ESCC" is the most common type of EC in developing countries, and the prognosis for the treatment of this cancer is very poor with the 5-year overall survival rate of only 20%-30%.

The term "immunotherapy" refers to the treatment of a subject with a disease or at risk of infection or disease recurrence by a method that includes inducing, enhancing, suppressing or otherwise modifying an immune response. The "treatment" or "therapy" of a subject refers to any type of intervention or process performed on the subject, or the administration of an active agent to the subject, with the purpose of reversing, alleviating, ameliorating, slowing or preventing the onset, progression, severity, or recurrence of symptoms, complications or conditions, or biochemical indicators associated with the disease.

A "programmed death receptor-1 (PD-1)" refers to an immunosuppressive receptor belonging to the CD28 family. PD-1 is expressed primarily on previously activated T cells *in vivo* and binds to two ligands, PD-L1 and PD-L2. The term "PD-1" used herein includes human PD-1 (hPD-1), variants, isotypes, and species homologs of hPD-1, and analogs having at least one common epitope with hPD-1.

A "therapeutically effective amount" or "therapeutically effective dose" of a medicament or therapeutic agent is any amount of the medicament that, when used alone or in combination with an additional therapeutic agent, protects a subject from the onset of a disease or promotes the regression of a disease as evidenced by a decrease in the severity of disease symptoms, an increase in the frequency and duration of disease symptom-free phase, or the prevention of injury or disability resulting from the affliction of the disease. The ability of a therapeutic agent to promote the regression of a disease can be assessed using a variety of methods known to those skilled in the art, such as in human subjects during clinical trials, in animal model systems that predict human efficacy, or by determining the activity of the agent in an *in vitro* assay.

A therapeutically effective amount of a medicament includes a "prophylactically effective amount" which is any amount of a medicament that, when administered alone or in combination with an anti-neoplastic agent, inhibits the development or recurrence of cancer to a subject at risk of developing cancer or a subject having cancer recurrence.

A "biotherapeutic agent" refers to a biological molecule, such as an antibody or fusion protein, that blocks ligand/receptor signaling in any biological pathway that supports tumor maintenance and/or growth or inhibits an anti-tumor immune response.

Unless otherwise specifically indicated, "CDR" used herein refers to a complementarity determining region of the immunoglobulin variable region defined using the Kabat numbering system.

An "anti-cancer agent" refers to any therapeutic agent that can be used in the treatment of cancer. Anti-cancer agents include, but are not limited to: alkylating agents, antimetabolites, kinase inhibitors, spindle poison phytoalkaloids, cytotoxic/anti-tumor antibiotics, photosensitizers, antiestrogens and selective estrogen receptor modulators, antiprogestins, aromatase inhibitors, CDK4/6 inhibitors, FGF/FGFR inhibitors and the like, and antisense oligonucleotides that inhibit the expression of genes involved in abnormal cell proliferation or tumor growth.

The term "about" refers to a value or composition within an acceptable error range for the particular value or composition, as determined by those of ordinary skill in the art, which depends in part on how the value or composition is measured or determined, i.e., the limitations of the measurement system. For example, "about" can refer to being within 1 or greater than 1 standard deviation according to practice in the art. Alternatively, "about" can refer to a range of up to 10% or 20% (i.e., ± 10% or ± 20%). For example, about 3 mg/kg can include any number between 2.7 mg/kg and 3.3 mg/kg (relative to 10%), and between 2.4 mg/kg and 3.6 mg/kg (relative to 20%). When a specific value or composition is provided herein, unless otherwise specifically stated, the meaning of "about" should be assumed to be within an acceptable error range of the specific value or composition.

A "therapeutic anti-PD-1 monoclonal antibody" refers to an antibody that specifically binds to the mature form of a specific PD-1 expressed on the surface of certain mammalian cells. Mature PD-1 does not have a secretory leader sequence, or leader peptide. The terms "PD-1" and "mature PD-1" are used interchangeably herein and are to be understood as the same molecule unless otherwise specifically defined, or clearly seen from the context.

A therapeutic anti-human PD-1 antibody or anti-hPD-1 antibody described herein refers to a monoclonal antibody that specifically binds to mature human PD-1.

A "framework region" or "FR" described herein refers to the immunoglobulin variable region excluding CDR regions.

An "isolated antibody or antigen-binding fragment thereof" refers to a molecule that is in a purified state, and in this case, is substantially free of other biomolecules, such as nucleic acids, proteins, lipids, carbohydrates, or other materials (such as cell debris or growth medium).

A "patient" or "subject" refers to any single subject in need of a medical procedure or participating in a clinical trial, epidemiological study, or serving as a control, including humans and mammals, such as horses, cows, dogs, or cats.

In the following paragraphs, various aspects of the present invention are described in further detail.

### Anti-PD-1 antibody

A "PD-1 antibody" refers to any chemical compound or biomolecule that binds to the PD-1 receptor, blocks the binding of PD-L1 expressed on cancer cells to PD-1 expressed on immune cells (T, B and NK cells), and preferably blocks the binding of PD-L2 expressed on cancer cells to PD-1 expressed on immune cells. Alternative nouns or synonyms for PD-1 and its ligands include: PDCD1, PD1, CD279 and SLEB2 for PD-1; PDCD1L1, PDL1, B7-H1, B7H1, B7-4, CD274 and B7-H for PD-L1; and PDCD1L2, PDL2, B7-DC and CD273 for PD-L2. In any of the therapy, medicament and use described herein for treating a human individual, the PD-1 antibody blocks the binding of human PD-L1 to human PD-1, and preferably blocks the binding of both human PD-L1 and PD-L2 to human PD1. The amino acid sequence of human PD-1 can be found at NCBI locus number: NP005009. The amino acid sequences of human PD-L1 and PD-L2 can be found at NCBI locus numbers: NP-054862 and NP-079515.

The anti-PD-1 antibody that can be used in any of the uses, therapies, medicaments and kits described herein includes a monoclonal antibody (mAb) or an antigen-binding fragment thereof that specifically binds to PD-1, and preferably specifically binds to human PD-1. The mAb can be a human antibody, a humanized antibody, or a chimeric antibody, and can include a human constant region. In some embodiments, the constant region is selected from human IgG1, IgG2, IgG3, and IgG4 constant regions, and in preferred embodiments, the constant region is a human IgG4 constant region.

In any one of the embodiments of the uses, therapies, medicaments and kits described herein, the PD-1 antibody is a monoclonal antibody or an antigen-binding fragment thereof comprising: (a) light chain CDRs comprising amino acid sequences set forth in SEQ ID NOs: 1, 2 and 3, and heavy chain CDRs comprising amino acid sequences set forth in SEQ ID NOs: 4, 5 and 6.

In any one of the embodiments of the uses, therapies, medicaments and kits described herein, the PD-1 antibody is a monoclonal antibody that specifically binds to human PD-1 and comprises: (a) a light chain variable region set forth in SEQ ID NO: 7, and (b) a heavy chain variable region set forth in SEQ ID NO: 8.

In any one of the embodiments of the uses, therapies, medicaments and kits described herein, the PD-1 antibody is a monoclonal antibody that specifically binds to human PD-1 and comprises: (a) a light chain variable region set forth in SEQ ID NO: 9, and (b) a heavy chain set forth in SEQ ID NO: 10.

In any one of the embodiments of the uses, therapies, medicaments and kits described herein, the PD-1 antibody is a monoclonal antibody or an antigen-binding fragment thereof, and the table A below provides a list of amino acid sequences of an exemplary anti-PD-1 mAb used in the uses, therapies, medicaments and kits described herein.

**Table A: light and heavy chain CDRs of an exemplary anti-human PD-1 antibody**

| LCDR1 | SEQ ID NO: 1 |
|---|---|
| LCDR2 | SEQ ID NO: 2 |
| LCDR3 | SEQ ID NO: 3 |
| HCDR1 | SEQ ID NO: 4 |
| HCDR2 | SEQ ID NO: 5 |
| HCDR3 | SEQ ID NO: 6 |

An example of anti-PD-1 antibodies that bind to human PD-1 and can be used in the uses, therapies, medicaments and kits described herein is described in WO2014206107. Human PD-1 mAbs that can be used as anti-PD-1 antibodies in the uses, therapies, medicaments and kits described herein include any one of the anti-PD-1 antibodies described in WO2014206107, including toripalimab (Toripalimab) (a humanized IgG4 mAb having the structure described in WHO Drug Information; 32(2):372-373 (2018) and comprising light and heavy chain amino acid sequences set forth in SEQ ID NOs: 9 and 10). In certain embodiments, anti-PD-1 antibodies that can be used in the uses, therapies, medicaments and kits described herein also include nivolumab and pamtuzumab that have been approved by FDA, and sintilimab that has been approved by NMPA, and SHR-1210 and tislelizumab that are in clinical stage, as well as any chemical compound or biomolecule that blocks the binding of PD-L1 expressed on cancer cells to PD-1 expressed on immune cells (T, B and NK cells) and preferably blocks the binding of PD-L2 expressed on cancer cells to PD-1 expressed on immune cells that are in preclinical and clinical stages.

In certain embodiments, anti-PD-1 antibodies that can be used in the uses, therapies, medicaments and kits described herein also include anti-PD-L1 monoclonal antibodies that specifically bind to PD-L1 to block the binding of PD-L1 to PD-1, such as atezolizumab, avelumab, durvalumab, or any chemical compound or biomolecule that specifically binds to PD-L1 to block the binding of PD-L1 to PD-1.

"PD-L1" expression or "PD-L2" expression described herein refers to any detectable expression level of a specific PD-L protein on the surface of a cell or a specific PD-L mRNA within a cell or tissue. PD-L protein expression can be detected in IHC analysis of tumor tissue sections or by flow cytometry using diagnostic PD-L antibodies. Alternatively, PD-L protein expression of tumor cells can be detected by PET imaging using a binding agent that specifically binds to a desired PD-L target (such as PD-L1 or PD-L2).

Methods for quantifying PD-L1 protein expression in IHC analysis of tumor tissue sections can be found in, but are not limited to, Thompson, R. H. et al, PNAS 101(49):17174-17179 (2004); Taube, J. M. et al, Sci Transl Med 4, 127ra37 (2012); and Toplian, S. L. et al, New Eng. J. Med. 366(26): 2443-2454 (2012), and the like.

In one method, a simple binary endpoint of positive or negative PD-L1 expression is adopted, where the positive PD-L1 expression is defined by the percentage of tumor cells showing histological evidence of cell surface membrane staining. The case where tumor cells on a tumor tissue section account for at least 1% of the total tumor cells is defined as PD-L1 positive.

In another method, PD-L1 expression in the tumor tissue section is quantified in tumor cells as well as in infiltrating immune cells. The percentage of tumor cells and infiltrating immune cells exhibiting membrane staining are quantified individually as < 1%, 1% to 50%, and subsequent 50% to 100%. For tumor cells, the PD-L1 expression is counted as negative if the score is < 1%, and positive if the score is > 1%.

In some embodiments of the present invention, the PD-L1 expression of ≥ 1% in the tumor tissue section of a subject does not achieve a better benefit compared to the PD-L1 expression of < 1% in the tumor tissue section of a subject. In some preferred embodiments, the cancer is a solid tumor. In some specific embodiments, the cancer is esophageal cancer. As a preferred embodiment, the cancer is ESCC.

The "RECIST 1.1 efficacy criteria" described herein refers to the definition of target injury and non-target injury described in Eisenhauver et al, E.A. et al, Eur. J Cancer 45:228-247(2009) in the context of the measured background.

The term "ECOG" score standard is an indicator of general health status and tolerance to treatment of patients from their physical strength. ECOG score standard for the physical strength is 0 points, 1 point, 2 points, 3 points, 4 points and 5 points. A score of 0 means that the motility is completely normal and has no difference from the motility before onset of disease. A score of 1 means that the person is free to walk and engages in light physical activities, including general housework or office work, but not in heavy physical activities.

A "sustained response" refers to a sustained therapeutic effect following cessation of treatment with a therapeutic agent or combination therapy described herein. In some embodiments, the sustained response has a duration that is at least the same as the duration of treatment or at least 1.5, 2.0, 2.5 or 3 times the duration of the treatment.

A "tissue section" refers to a single portion or piece of a tissue sample, such as a tissue slice cut from a sample of normal tissue or a tumor.

As used herein, "treating" cancer refers to administering a treatment regimen described herein (e.g., administration of an anti-PD-1 antibody, or administration of a combination therapy of an anti-PD-1 antibody with a CDK4/6 inhibitor or an FGF/FGFR inhibitor) to a subject with or diagnosed with cancer to achieve at least one positive therapeutic effect (e.g., a decrease in cancer cell number, a decrease in tumor volume, a reduction in the rate of cancer cell infiltration into peripheral organs, or a reduction in the rate of tumor metastasis or tumor growth). Positive therapeutic effects in cancer can be measured in a variety of ways (see W. A. Weber, J. Nucl. Med., 50:1S-10S (2009)). For example, T/C ≤ 42% for tumor growth inhibition is the minimum level of anti-tumor activity according to the NCI criteria. It is considered that T/C (%) = median treated tumor volume/median control tumor volume × 100. In some embodiments, the therapeutic effect achieved by the combination of the present invention is any one of PR, CR, OR, PFS, DFS and OS. PFS (also called "time to tumor progression") refers to the length of time during and after treatment during which cancer does not grow, and includes the amount of time a patient experiences CR or PR and the amount of time a patient experiences SD. DFS refers to the length of time during and after treatment during which a patient remains disease-free. OS refers to an extension of life expectancy compared to an initial or untreated individual or a patient. In some embodiments, the response to the combination of the present invention is any one of PR, CR, PFS, DFS, OR OS, assessed using RECIST 1.1 efficacy criteria. The treatment regimen of the combination of the present invention effective in treating a cancer patient may vary depending upon a variety of factors such as the disease state, age, weight of the patient and the ability of the therapy to elicit an anti-cancer response in the subject. Embodiments of the present invention may not achieve an effective positive therapeutic effect in each subject, but should be effective and achieve a positive therapeutic effect in a statistically significant number of subjects.

The terms "mode of administration" and "dosing regimen" are used interchangeably and refer to the dosage and time of use of each therapeutic agent in the combination of the present invention.

A "tumor", when applied to a subject diagnosed with or suspected of having cancer, refers to a malignant or potentially malignant neoplasm or tissue mass of any size, and includes primary tumors and secondary neoplasms. Solid tumors typically do not contain abnormal growth or mass of tissue in cysts or fluid areas. Different types of solid tumors are named for the cell type from which they are formed. Examples of solid tumors are sarcomas, carcinomas and lymphomas. Hematologic cancers typically do not form solid tumors.

"Tumor burden" refers to the total amount of tumor mass distributed throughout the body. Tumor burden refers to the total number of cancer cells or the total size of the tumor throughout the body. Tumor burden can be determined by a variety of methods known in the art, such as measuring the size of a tumor using calipers after the tumor is removed from a subject, or using imaging techniques (e.g., ultrasound, bone scanning, computed tomography (CT), or magnetic resonance imaging (MRI) scanning) when the tumor is *in vivo.*

The term "tumor size" refers to the total size of a tumor, which can be measured as the length and width of the tumor. Tumor size can be determined by a variety of methods known in the art, such as measuring the size of a tumor using calipers after the tumor is removed from a subject, or using imaging techniques (e.g., bone scanning, ultrasound, CT, or MRI scanning) when the tumor is *in vivo.*

The term "tumor mutation burden (TMB)" refers to the total number of gene coding errors, base substitutions, gene insertion or deletion errors detected in a somatic cell per million bases. In some embodiments of the present invention, tumor mutation burden (TMB) is estimated by analysis of somatic mutations, including coding base substitutions and the megabase insertions of the panel sequences studied. In some embodiments of the present invention, the tumor mutation burden (TMB) of ≥ 12 mutations/Mb of a subject does not achieve a better benefit compared to the tumor mutation burden (TMB) of < 12 mutations/Mb of a subject. In some preferred embodiments, the cancer is a solid tumor. In some specific embodiments, the cancer is esophageal cancer. As a preferred embodiment, the cancer is ESCC.

The term "gene amplification" refers to a process in which the copy number of a gene encoded by a specific protein is increased selectively while the number of the other genes is not increased proportionally. Under natural conditions, the gene amplification is achieved by excising repeated sequences of a gene from the chromosome and then performing extrachromosomal replication in a plasmid, or by transcribing all the repeated sequences of ribosomal RNA to give RNA transcripts and then transcribing them to give additional copies of the original DNA molecule. In the present invention, gene sequencing analysis is disclosed in some examples. In some embodiments of the present invention, the subject described herein has certain unique gene amplifications. In some preferred embodiments, the subject has a gene amplification of the chromosome 11q13 region. In some further preferred embodiments, the subject has a CDK4/6 gene amplification; in some further preferred embodiments, the subject has an FGF3/4/19 gene amplification. As a preferred embodiment, the esophageal cancer subject has a CDK4/6 gene amplification. As a preferred embodiment, the esophageal cancer subject has an FGF3/4/19 gene amplification. In some embodiments, the tumor patient has a gene amplification of the chromosome 11q13 region, suggesting a better therapeutic effect for the treatment with (a) an inhibitor of CDK4/6 and/or an FGF/FGFR inhibitor alone, or (b) an anti-PD-1 antibody or an antigen-binding fragment thereof in combination with one of or a combination of more of a CDK4/6 inhibitor and/or an FGF/FGFR inhibitor. In some embodiments, the tumor patient has a gene amplification of the chromosome 11q13 region, suggesting a better therapeutic effect for the treatment with (a) the administration of an anti-PD-1 antibody or an antigen-binding fragment thereof alone, or (b) the administration of an anti-PD-1 antibody or an antigen-binding fragment thereof in combination with one of or a combination of more of a CDK4/6 inhibitor and/or an FGF/FGFR inhibitor.

The term "CDKs (cyclin-dependent kinases)" is a group of serine/threonine protein kinases, which drive the cell cycle by phosphorylation of serine/threonine proteins through synergistic interaction with cyclin and are important factors in cell cycle regulation. The CDK family has 8 types, including CDK1-8, each of which binds to a different types of cyclins to form a complex that regulates the transition of cells from G1 phase to S phase or G2 phase to M phase and the exiting from M phase. Currently, CDK4/6 inhibitors that have been approved by the FDA for marketing mainly include ribociclib and palbociclib, and dozens of CDK4/6 inhibitors in clinical research stage.

The term "FGF" is a family of fibroblast growth factor proteins, which has a total of 23 family members, namely FGF1-23. FGFs can be divided into three types according to their different mechanisms of action: endocrine (FGF15/19/21/23), paracrine (FGF1-10, FGF16-18, FGF20, FGF22), and cytocrine (FGF11/12/13/14). Paracrine FGFs regulate biological activity by using HS as a cofactor, and specifically binding to FGF receptors (fibroblast growth factor receptors, FGFRs) on cell surfaces. FGFRs mainly include 4 types: FGFR1, FGFR2, FGFR3 and FGFR4. Currently, there is no FGF/FGFR inhibitor on the market, VEGFR/PDGFR/FGFR inhibitor from Boehringer Ingelheim nintedanib for treating liver cancer, non-small cell lung cancer and idiopathic fibrosis received breakthrough therapy designation from FDA in 2014, and lucitanib is currently in clinical stage in China.

The term "immunohistochemistry (IHC)" refers to a method for determining antigens (polypeptides and proteins) in tissue cells by developing chromogenic agents (fluoresceins, enzymes, metal ions, isotopes) that label antibodies through chemical reaction based on the principle that antigens specifically binds to antibodies, and performing localized, qualitative and relatively quantitative studies on those antigens. In some embodiments of the present invention, a tumor tissue sample from a subject is tested for PD-L1 expression prior to treatment with an anti-PD-1 antibody by staining the anti-human PD-L1 antibody SP142 from Roche (Cat No: M4422). In some embodiments, tumor cells with membrane staining ≥ 1% are defined as PD-L1 positive.

### Pharmaceutical composition and dosage

The therapeutic agent of the present invention can constitute a pharmaceutical composition, such as a pharmaceutical composition comprising the anti-PD-1 antibody described herein or/and an additional anti-cancer agent other than the anti-PD-1 antibody, and an additional pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Preferably, the carrier suitable for the composition comprising the anti-PD-1 antibody is suitable for intravenous, intramuscular, subcutaneous, parenteral, spinal, or epidermal administration, such as by injection or infusion, while the carrier suitable for the composition comprising an additional anti-cancer agent is suitable for parenteral administration, such as oral administration. The pharmaceutical composition of the present invention can contain one or more pharmaceutically acceptable salts, antioxidants, water, non-aqueous carriers, and/or adjuvants such as preserving agent, wetting agent, emulsifying agent, and dispersing agent.

The dosage regimen is adjusted to provide the optimal desired response, such as the maximum therapeutic response and/or the minimum adverse effect. The dose of the anti-PD-1 antibody, when administered in combination with another anti-cancer agent, can range from about 0.01 mg/kg body weight to about 20 mg/kg body weight, about 0.1 mg/kg body weight to about 10 mg/kg body weight, or can be a fixed dose of 120 mg, 240 mg, 360 mg or 480 mg. For example, the dose can be about 0.1 mg/kg body weight, about 0.3 mg/kg body weight, about 1 mg/kg body weight, about 2 mg/kg body weight, about 3 mg/kg body weight, about 5 mg/kg body weight, or about 10 mg/kg body weight. Dosing regimens are generally designed to achieve an exposure that results in sustained receptor occupancy (RO) based on the typical pharmacokinetic properties of Ab. A representative dosing regimen can be performed about once a week, about once every two weeks, about once every three weeks, about once every four weeks, about once a month, or longer. In some embodiments, the anti-PD-1 antibody is administered to an individual about once every two weeks.

The dosing schedule of the additional anti-cancer agent varies for different medicaments.

In some embodiments of the present invention, the dosing schedule of the CDK4/6 inhibitor or FGF/FGFR inhibitor varies for different subtypes. For the combination therapy of an anti-PD-1 antibody with a CDK4/6 inhibitor or an FGF/FGFR inhibitor, in some embodiments, the CDK4/6 inhibitor or the FGF/FGFR inhibitor is administered at its approved or recommended dose, and the treatment is continued until clinical effects are observed or until unacceptable toxicity or disease progression occurs.

### Method of the present invention

The present invention relates to a methods for treating a cancer patient, comprising administering to the cancer patient (a) a therapeutically effective amount of an anti-PD-1 antibody or an antigen-binding fragment thereof alone, or (b) a therapeutically effective amount of an anti-PD-1 antibody or an antigen-binding fragment thereof in combination with optionally one of or a combination of more of additional anti-cancer agents other than the anti-PD-1 antibody and the antigen-binding fragment thereof.

In certain embodiments, a cancer patient suitable for the administration of (a) a therapeutically effective amount of an anti-PD-1 antibody or an antigen-binding fragment thereof alone, or (b) a therapeutically effective amount of an anti-PD-1 antibody or an antigen-binding fragment thereof in combination with optionally one of or a combination of more of additional anti-cancer agents other than the anti-PD-1 antibody and the antigen-binding fragment thereof described herein, is preferably a cancer patient in which the gene amplification of the chromosome 11q13 region is not detected. Therefore, in some embodiments, the method for treating cancer also comprises sequencing the cancer patient.

In certain embodiments, the present invention relates to a method for treating a cancer patient, comprising administering to the cancer patient (a) a therapeutically effective amount of an additional anti-cancer agent alone, or (b) a therapeutically effective amount of an anti-PD-1 antibody or an antigen-binding fragment thereof in combination with optionally one of or a combination of more of an additional anti-cancer agent other than the anti-PD-1 antibody.

In certain embodiments, a cancer patient suitable for the administration of (a) a therapeutically effective amount of an additional anti-cancer agent, or (b) a therapeutically effective amount of an anti-PD-1 antibody or an antigen-binding fragment thereof in combination with optionally one of or a combination of more of additional anti-cancer agents other than the anti-PD-1 antibody described herein, is preferably a cancer patient in which the gene amplification of the 11q13 region is detected, particularly a cancer patient having a CDK4/6 gene amplification or an FGF3/4/19 gene amplification. Therefore, in some embodiments, the method for treating cancer also comprises sequencing the cancer patient.

In certain embodiments, the additional anti-cancer agent is a therapeutically effective amount of a CDK4/6 inhibitor and/or an FGF/FGFR inhibitor.

In certain embodiments, the present invention provides a method for treating an individual/patient with esophageal cancer. In some embodiments, the method comprises administering to the individual a therapeutically effective dose of the combination of: (a) an Ab or an antigen-binding fragment thereof that specifically binds to PD-1 receptor and inhibits PD-1 activity; and (b) another anti-cancer therapy. In one embodiment, the method comprises administering to the individual an effective amount of a combination of: (i) a standard therapy for treating esophageal cancer, as disclosed elsewhere herein, or (ii) an additional anti-cancer agent. In some embodiments, the additional anti-cancer agent is selected from a CDK4/6 inhibitor and/or an FGF/FGFR inhibitor. Since the most common histological subtype of esophageal cancer in esophageal cancer patients in developing countries is esophageal squamous cell carcinoma (ESCC), in some embodiments, the esophageal cancer is esophageal squamous cell carcinoma (ESCC). In certain embodiments, the method also comprises sequencing a patient prior to administering the therapeutic agent; based on the sequencing results, an individual not having an amplification of the chromosome 11q13 region is administered with an anti-PD-1 antibody or an antigen-binding fragment thereof alone, optionally in combination with one of or a combination of more of a CDK4/6 inhibitor and/or an FGF/FGFR inhibitor; an individual having an amplification of the chromosome 11q13 region, in particular having a CDK4/6 gene amplification or an FGF3/4/19 gene amplification, is administered with one of or a combination of more of a CDK4/6 inhibitor and/or an FGF/FGFR inhibitor alone, optionally in combination with an anti-PD-1 antibody or an antigen-binding fragment thereof.

### Anti-PD-1 antibody suitable for the method of the present invention

The antibody PD-1 antibody suitable for the method of the present invention has an immunosuppressive effect achieved by binding to PD-1 with high specificity and affinity, blocking the binding of PD-L1/2 to PD-1 and inhibiting PD-1 signal transduction. In any of the therapies disclosed herein, the anti-PD-1 antibody includes an antigen-binding portion or fragment that binds to the PD-1 receptor and exhibits functional properties similar to an intact Ab in inhibiting ligand binding and upregulating the immune system. In some embodiments, the anti-PD-1 antibody or the antigen-binding fragment thereof is an anti-PD-1 antibody or an antigen-binding fragment thereof that cross-competes for binding to human PD-1 with toripalimab. In other embodiments, the anti-PD-1 antibody or the antigen-binding fragment thereof is a chimeric, humanized or human Ab or an antigen-binding fragment thereof. In certain embodiments for treating a human individual, the Ab is a humanized Ab.

In some embodiments, the anti-PD-1 antibody or the antigen-binding fragment thereof comprises a human IgG1 or IgG4 heavy chain constant region. In some embodiments, the sequence of the IgG4 heavy chain constant region of the anti-PD-1 antibody or the antigen-binding fragment thereof comprises the S228P mutation that replaces a serine residue in the hinge region with a proline residue that is typically present at the corresponding position of an antibody of IgG1 isotype. In certain embodiments of any of the treatment methods described herein comprising administering an anti-PD-1 antibody, the anti-PD-1 antibody is toripalimab. In some embodiments, the anti-PD-1 antibody is selected from humanized antibodies 38, 39, 41 and 48 described in WO2014206107. In some embodiments, the anti-PD-1 antibody is as described in the "Anti-PD-1 antibody" section above.

### CDK4/6 inhibitors suitable for the method of the present invention

In some embodiments of the present invention for treating tumors, the additional anti-cancer agent, i.e., an anti-cancer agent other than the anti-PD-1 antibody administered alone or in combination with the anti-PD-1 antibody, is a CDK4/6 inhibitor. CDK4/6 inhibitors currently approved by the FDA for marketing mainly includes Verzenio (abemaciclib) from Eli Lilly, which is mainly used for treating hormone receptor (HR)-positive and human epidermal growth factor receptor 2 (HER2)-negative adult patients with advanced or metastatic breast cancer having disease progression after receiving endocrine therapy; Kisqali (ribociclib) from Novartis, which is used in combination with aromatase inhibitor as a first-line treatment for HR-positive and HER2-negative postmenopausal female patients with advanced metastatic breast cancer; and Ibrance (palbociclib) from Pfizer, which is used in combination with letrozole for treating estrogen receptor (ER)-positive and human epidermal growth factor receptor 2 (HER2)-negative postmenopausal women with metastatic breast cancer.

### FGF/FGFR inhibitors suitable for the method of the present invention

In some embodiments of the present invention for treating tumors, the additional anti-cancer agent, i.e., an anti-cancer agent other than the anti-PD-1 antibody administered alone or in combination with the anti-PD-1 antibody, is an FGF/FGFR inhibitor. Currently, there is no FGF/FGFR inhibitor on the market, VEGFR/PDGFR/FGFR inhibitor from Boehringer Ingelheim nintedanib for treating liver cancer, non-small cell lung cancer and idiopathic fibrosis received breakthrough therapy designation from FDA in 2014, and lucitanib is currently in clinical stage in China.

### Uses, therapies, medicaments and kits

In one aspect of the present invention, provided is a method for treating cancer in an individual, comprising administering to the individual a combination therapy comprising an anti-PD-1 antibody or an antigen-binding fragment thereof and one of or a combination of more of a CDK4/6 inhibitor and an FGF/FGFR inhibitor.

The combination therapy can also comprise one or more additional therapeutic agents. The additional therapeutic agent can be a chemotherapeutic agent or a biotherapeutic agent other than a CDK4/6 inhibitor and an FGF/FGFR inhibitor.

According to standard pharmaceutical practice, each therapeutic agent in the combination therapy of the present invention can be administered alone or in a pharmaceutical composition comprising the therapeutic agent and one or more pharmaceutically acceptable carriers, excipients, and diluents.

Each therapeutic agent in the combination therapy of the present invention can be administered simultaneously, concurrently or sequentially in any order. The therapeutic agents in the combination therapy are administered in different dosage forms, such as one medicament being a tablet or a capsule and the other medicament being a sterile liquid, and/or at different dosing times, such as the chemotherapeutic agent being administered at least daily and the biologic therapeutic agent being administered infrequently, such as once every week, or every two weeks or every three weeks.

In some embodiments, the CDK4/6 inhibitor and the FGF/FGFR inhibitor are administered prior to the administration of the anti-PD-1 antibody, while in other embodiments, the CDK4/6 inhibitor and the FGF/FGFR inhibitor are administered after the administration of the anti-PD-1 antibody.

In some embodiments, at least one of the therapeutic agents in the combination therapy is administered using the same dosing regimen (dose, frequency, duration of treatment) when the medicaments are used as a monotherapy for treating the same cancer.

Each small molecule therapeutic agent in the combination therapy described herein can be administered orally or parenterally (such as by intravenous, intramuscular, intraperitoneal, subcutaneous, rectal, topical, or transdermal routes of administration).

The combination therapy described herein can be administered before or after surgery and can be administered before, during or after radiotherapy.

In some embodiments, the combination therapy described herein is administered to a patient who has not received prior treatment with a biologic agent or a chemotherapeutic agent. In other embodiments, the combination therapy is administered to a patient who fails to achieve a sustained response after the treatment with a biologic agent or a chemotherapeutic agent.

The combination therapy of the present invention can be used in the treatment of tumors found by palpation or by imaging techniques known in the art, such as MRI, ultrasound or CAT scanning.

The dosage regimens for the combination therapy of the present invention are selected depending on several factors including, but not limited to, serum or tissue conversion rate, degree of symptoms, immunogenicity, and the accessibility of the target cell, tissue, and organ of the treated individual. Preferably, the dosing regimen is designed to deliver the maximum amount of each therapeutic agent to the patient based on an acceptable degree of side effects. Therefore, the dose and dosing frequency of each of the biotherapeutic agents and chemotherapeutic agents in the combination therapy depends on the specific therapeutic agent, the severity of the cancer being treated and the characteristics of the patient.

The anti-PD-1 antibody or the antigen-binding fragment thereof described herein and one of or a combination of more of a CDK4/6 inhibitor and an FGF/FGFR inhibitor can be provided as a kit comprising a first container, a second container and a package insert.

The first container contains at least one dose of a medicament comprising the anti-PD-1 antibody or the antigen-binding fragment thereof, the second container contains at least one dose of a medicament comprising one of or a combination of more of a CDK4/6 inhibitor and an FGF/FGFR inhibitor, and the package insert or label contains instructions for use of the medicament in the treatment of cancer. The kit can further comprise other materials that can be used in the administration of a medicament, such as diluents, filter paper, IV bags and threads, needles and syringes.

### Method for predicting therapeutic effect of anti-PD-1 antibody on cancer

The method for predicting the effect of the anti-PD-1 antibody or the antigen-binding fragment thereof, particularly toripalimab, administered alone or in combination for treating cancer in an individual described herein comprise sequencing the individual prior to treatment. In some embodiments, the cancer is esophageal cancer; in other preferred embodiments, the cancer is ESCC.

In one embodiment, the prediction method described herein is a gene test of an individual prior to treatment to assess the presence or absence of a gene amplification of the chromosome 11q13 region. In one embodiment, the individual does not have the gene amplification of the chromosome 11q13 region. In another embodiment, the individual has the gene amplification of the chromosome 11q13 region. In one embodiment, the individual has CCND1 gene amplification and/or FGF/FGFR gene amplification. In one embodiment, the individual has CDK4/6 gene amplification and/or FGF3/4/19 gene amplification.

The present invention also includes a method for predicting the therapeutic effect of a tumor patient administered with the anti-PD-1 antibody or the antigen-binding fragment thereof alone or in combination using genes of the chromosome 11q13 region. The absence of the gene amplification of the chromosome 11q13 region indicates that the tumor patient is suitable for the treatment with the administration of the anti-PD-1 antibody or the antigen-binding fragment thereof alone or in combination.

In certain embodiments, the present invention also provides use of a detection reagent of biomarkers in the preparation of a kit for predicting the therapeutic effect of the anti-PD-1 antibody on cancer. Such reagents include, for example, those for detecting the presence or absence of the gene amplification of the chromosome 11q13 region in genes of an individual. Preferably, such agents include, for example, those for detecting the presence or absence of CCND1 gene amplification and/or FGF/FGFR gene amplification in genes of an individual. More preferably, such reagents include, for example, those for detecting the presence or absence of CDK4/6 gene amplification and/or FGF3/4/19 gene amplification in genes of an individual.

The present invention also includes use of the reagent for detecting genes of the chromosome 11q13 region in the preparation of a kit for predicting the therapeutic effect of the anti-PD-1 antibody on cancer. Such reagents include, but are not limited to, those conventionally used in assays, including but not limited to, primers, probes, reagents required for PCR, and the like.

### Abbreviation

The following abbreviations are used throughout the description and examples of the present invention:
- BID: One dose, twice a day
- CDR: Complementarity determining region
- FR: Framework region
- IgG: Immunoglobulin G
- IHC: Immunohistochemistry
- WES: Whole exome sequencing
- PBMC: Peripheral blood mononuclear cell
- OR: Overall response
- ORR: Objective response rate
- OS: Overall survival
- PD: Progression of disease
- PFS: Progression-free survival
- DFS: Disease-free survival
- DCR: Disease control rate
- PR: Partial response
- CR: Complete response
- SD: Stable disease
- DLT: Dose-limiting toxicity
- MTD: Maximum tolerated dose
- AE: Adverse event
- Q2W: One dose every 2 weeks
- QD: One dose everyday
- TRAE: Treatment-related adverse event
- SAE: Serious adverse event
- TMB: Tumor mutation burden
- EC: Esophageal cancer
- ESCC: Esophageal squamous cell carcinoma
- LDH: Lactate dehydrogenase
- ECOG: Eastern cooperative oncology group

The present invention is further illustrated by the following examples, which should not be construed as limiting the present invention. The contents of all references cited throughout this application are expressly incorporated herein by reference.

### EXAMPLES

### Example 1.Clinical study on anti-PD-1 antibody alone for treating tumors

Enrollment criteria: eligible subjects must (1) be between 18 and 75 years old, (2) be histologically and/or cytologically confirmed as having advanced and/or metastatic ESCC, (3) be patients with advanced ESCC having received or at least received one treatment with still progressing (including but not limited to chemotherapy or radiotherapy), (4) have an ECOG score of 0 or 1.

Subjects must have an assessable lesion according to RECIST v1.1 criteria, no other prior or concurrent malignancies, no any active autoimmune disease or history of autoimmune disease, no concomitant disease requiring long-term immunosuppressive drug therapy, no prior treatment with anti-CTLA4, anti-PD-1 or anti-PD-L1 antibodies, no active hepatitis B or C virus infection, no pregnancy or not in lactation, and no anti-tumor therapy, radiotherapy or any surgical treatment for the first 4 weeks prior to enrollment. Demographic data of the enrolled subjects are shown in Table 1.

**Table 1: Demographic data of the enrolled subjects**

| Characteristics | | Esophageal cancer (TC) (n = 60) |
|---|---|---|
| Average age, year (range) | | 60.5 (42.0, 73.0) |
| Sex | Male | 53 (88.3) |
| | Female | 7 (11.7) |
| ECOG state | 0 | 5 (8.3) |
| | 1 | 54 (90.0) |
| | Unknown | 1 (1.7) |
| Liver metastasis | No | 44 (73.3) |
| | Yes | 15 (25.0) |
| | Unknown | 1 (1.7) |
| Baseline LDH (IU/L) | Normal | 45 (75.0) |
| | Abnormal, clinically insignificant | 12 (20.0) |
| | Abnormal, clinically significant | 3 (5.0) |
| Prior treatment | No | 0 |
| | 1L | 13 (21.7) |
| | 2L | 20 (33.3) |
| | 3L+ | 27 (45.0) |
| PD-L1 results* | Negative | 39 (65.0) |
| | Positive | 19 (31.7) |
| | Not detected | 2(3.3) |

| | | |
|---|---|---|
| Note: *positive is defined as the presence of ≥ 1% tumor cells or immune cells by SP142 IHC staining; abbreviations: ECOG, eastern cooperative oncology group; LDH, lactate dehydrogenase. | | |

Test drug: the anti-PD-1 antibody toripalimab (Toripalimab) (WO2014206107).

The anti-PD-1 antibody dose groups for this study: 3 mg/kg dose group at first. After enrollment, subjects will receive treatment every 2 weeks (Q2W) for a 4-week cycle, until disease progression, onset of intolerable toxicity, withdrawal of consent by the subject, no further benefit at the discretion of the investigator, or death.

Clinical design:
This is an open, multi-center clinical phase Ib/II trial divided into 8 independent cohorts, and the content of the present invention focuses on accessing the results of cohort 2, so as to evaluate the safety, tolerability, and anti-tumor activity of the anti-PD-1 antibody in the treatment of chemotherapy-refractory advanced ESCC.

From March 9, 2017 to August 24, 2017, a total of 60 subjects with advanced ESCC were enrolled, of whom 59 subjects were treated with toripalimab and 1 subject withdrew consent prior to treatment.

The planned dose was 3 mg/kg, Q2W.

### 1.1 Safety study

As of December 31, 2018, 16 months after the last patient was enrolled, 59 patients receiving at least one dose of toripalimab were included in the safety analysis. Treatment-related adverse events (TRAEs) were observed in 56 of 59 patients (94.9%), but most of the TRAEs were grade 1 or 2 (Table 2). Common TRAEs include weight loss (18.6%), anemia (18.6%), loss of appetite (18.6%), fever (16.9%), cough (16.9%), leukopenia (15.3%), AST elevation (13.6%), hypothyroidism (13.6%), ALT elevation (11.9%), fatigue (11.9%), nausea (11.9%), total bilirubin increase (10.2%), low hemoglobin counts (10.2%), and constipation (10.2%) (Table 2). Grade 3 and higher TRAEs were observed in 19 (32.2%) patients. Of them, 11 (18.6%) patients developed grade 3 TRAEs, including 2 cases of anemia, 2 cases of hyponatremia, 1 case of hypertension, 1 case of anorexia, 1 case of dysphagia, 1 case of esophageal stenosis, 1 case of fatigue, 1 case of hydrocardia, 1 case of hypercalcemia, 1 case of leukopenia, 1 case of upper respiratory infection and 1 case of neck infection. Grade 4 TRAEs were observed in 2 (3.4%) patients, including 1 case of hyperuricemia and 1 case of paraplegia. All the six treatment-related deaths were likely unrelated to the treatment, including 1 case of cachexia, 2 cases of pneumonia and 3 cases of unknown etiology. 14 (23.7%) patients were permanently discontinued due to TRAE; 6 (10.2%) patients experienced dose discontinuation due to TRAE. It can be seen that toripalimab has controlled safety in ESCC patients.

**Table 2. Common treatment-related adverse events in the cohort (> 10%**

| | Esophageal cancer patients receiving toripalimab (n = 59) | | | | | |
|---|---|---|---|---|---|---|
| | Level 1 n(%) | Level 2 n(%) | Level 3 n (%) | Level 4 n(%) | Level 5 n (%) | Total n(%) |
| Treatment-related adverse events | 13(22.0) | 24(40.7) | 11(18.6) | 2(3.4) | 6(10.2) | 56(94.9) |
| Weight loss | 8(13.6) | 3(5.1) | 0 | 0 | 0 | 11(18.6) |
| Loss of appetite | 6(10.2) | 4(6.8) | 1(1.7) | 0 | 0 | 11(18.6) |
| Anemia | 6(10.2) | 2(3.4) | 3(5.1) | 0 | 0 | 11(18.6) |
| Fever | 8(13.3) | 2(3.4) | 0 | 0 | 0 | 10(16.9) |
| Cough | 6(10.2) | 4(6.8) | 0 | 0 | 0 | 10(16.9) |
| Leukopenia | 6(10.2) | 3(5.1) | 0 | 0 | 0 | 9(15.3) |
| AST elevation | 8(13.6) | 0 | 0 | 0 | 0 | 8(13.6) |
| Hypothyroidism | 5(8.5) | 3(5.1) | 0 | 0 | 0 | 8(13.6) |
| ALT elevation | 7(11.9) | 0 | 0 | 0 | 0 | 7(11.9) |
| Nausea | 5(8.5) | 2(3.4) | 0 | 0 | 0 | 7(11.9) |
| Fatigue | 6(10.2) | 1(1.7) | 0 | 0 | 0 | 7(11.9) |
| Total bilirubin increase | 6(10.2) | 0 | 0 | 0 | 0 | 6(10.2) |
| Low hemoglobin counts | 3(5.1) | 3(5.1) | 0 | 0 | 0 | 6(10.2) |
| Constipation | 5(8.5) | 1(1.7) | 0 | 0 | 0 | 6(10.2) |

### 1.2 Anti-tumor activity study

As of December 31, 2018, 67.8% (40/59) of the subjects had died. 5.1% (3/59) discontinued follow-up, 11.9% (7/59) withdrew consent, 74.5% (44/59) discontinued treatment, and 8.5% (5/59) were still under study.

The median duration of treatment was 3.5 months (between 0.1 and 19.1 months).

As of the data cutoff date, 1 case of CR, 10 cases of PR (including 2 cases of undiagnosed PR) and 17 cases of SD in 59 patients were assessed by investigators using RECIST v1.1. The optimal response rate was 18.6% (95% CI 9.7 to 30.9) and the DCR was 47.5% (95% CI 34.3 to 60.9). CR patients had received 1-line chemotherapy and no radiotherapy. 10 PR patients had received radiotherapy, of whom 4 patients had received 2 prior systemic chemotherapies and the other 6 patients had received 3 or 4 prior chemotherapies.

### Progression free survival and overall survival

The ORR confirmed was 15.3% (95% CI 7.2 to 27.0). The mean response time was 1.8 months. The median duration of response was 11.2 months. An arbitrary size reduction in target lesions relative to baseline was observed in 25 (42.4%) subjects (FIG.s 1A and 1B). The median PFS was 2.1 months and the median OS was 6.9 months (FIG.s 1C and 1D).

### Example 2. Study on the corresponding relation between PD-L1 expression and therapeutic effect of anti-PD-1 antibody on tumors

Subjects were subjected to an archived or fresh tumor biopsy prior to treatment with toripalimab in the clinical trial described in Example 1. PD-L1 was detected in formalin-fixed paraffin-embedded (FFPE) tumor tissue samples by immunohistochemistry (IHC) methods and verified on the VENTANA Benchmark Ultra system at the clinical center laboratory (Q2 laboratory, Beijing). The immunohistochemistry detection of PD-L1 was performed using the Spring Bioscience (Roche) rabbit anti-human PD-L1 monoclonal antibody (clone SP142, Cat No: M4422). The expression of PD-L1 on tumor cells (TCs) and tumor infiltrating immune cells (ICs) was assessed by certified pathologists using stained tumor tissue. The PD-L1 positive status is defined as the presence of tumor cells with membrane staining intensity ≥ 1% or the presence of PD-LI staining intensity of tumor infiltrating immune cells covering ≥ 1% of related, continuous peri-cancerous stromal tumor area in the tumor occupied by tumor cells.

PD-L1 expression determination was performed on tumor biopsy samples from 57 subjects in Example 1. 19 (33.3%) positive PD-L1 expression and 38 (66.7%) negative PD-L1 expression samples were identified (FIG. 2A).

PD-L1 positive was defined as the presence of ≥ 1% positive tumor cells (TCs) or immune cells (ICs) by SP142 IHC staining.

In this study, there was no significant difference in ORR (15.8% vs. 18.4%) or OS (6.7 months vs. 6.9 months) between PD-L1 positive patients and PD-L1 negative subjects (FIGs. 2A, 2C and 2D). The median PFS of PD-L1 positive subjects was numerically superior to that of PD-L1 negative subjects (3.4 months vs. 2.0 months), although the difference was not statistically significant (p = 0.85) (FIG. 2C).

Only 5 (8.8%) subjects had more than 5% positive PD-L1 expression in the tumor biopsies in this study (FIG. 2A). Clinical response was better in subjects with more than 5% PD-L1 expression than in patients with low or no PD-L1 expression (40% ORR and 100% DCR in PD-L1 positive subjects vs. 15.4% ORR and 42.3% DCR in negative subjects). The difference in ORR was not statistically significant (*p* = 0.21), and the difference in DCR was statistically significant (*p* = 0.019).

### Example 3. Study on the corresponding relation between tumor mutation burden (TMB) and therapeutic effect of anti-PD-1 antibody on tumors

During the clinical trial described in Example 1, tumor biopsy samples from 50 subjects were subjected to whole exome sequencing (WES). TMB was determined by analyzing somatic mutations within coding regions of the human genome. Valid TMB results were obtained in 47 patients (Table 3).

In this study, patients with ESCC had a generally lower TMB. None of the biopsy samples had more than 20 mutations per million base pairs, and only 13 biopsy samples had 10-20 mutations per million base pairs.

Patients with TMB equal to or greater than 12 mutations/Mb (n = 11) had similar ORR (18.2% vs. 19.4%) as patients with TMB less than 12 mutations/Mb (n = 36) taking 12 mutations/Mb as a cutoff value (FIG. 2B). The group with high TMB (≥ 12) had numerically better PFS (4.0 months vs. 1.9 months) and OS (11.5 months vs. 6.7 months) than the group with low TMB (< 12), although the difference was not statistically significant (FIGs. 2E and 2F).

**Table 3. Corresponding relation between TMB and therapeutic effect of anti-PD-1 antibody on tumors**

| **Subject ID** | **Therapeutic effect** | **ECOG** | **% TC PD-L1** | **% IC PD-L1** | **Age** | **Sex** | **Tumor burden (mm)** | **Prior treatment** | **Liver metastasis** | **LDH (U/L)** | **TMB Muts/Mb** | **11q13 amplification** | **OS** | **State** | **PFS** | **State** |
|---|---|---|---|---|---|---|---|---|---|---|---|---|---|---|---|---|
| 1001075 | PD | 1 | 0.0 | 0.0 | 65.0 | Male | 33 | 2L | N | 273.6 | NA | NA | 203 | 0 | 50 | 1 |
| 1001087 | PD | 1 | 0.0 | 1.0 | 42.0 | Male | 40 | 2L | N | 150.2 | 3.1 | 0 | 106 | 0 | 50 | 1 |
| 1001093 | uPR | 1 | 0.0 | 1.0 | 46.0 | Male | 37 | 2L | Y | 164.1 | 4.2 | 0 | 242 | 1 | 112 | 1 |
| 1001109 | PD | 0 | 0.0 | 0.5 | 55.0 | Male | 82 | 3L+ | N | 191.8 | 4.1 | 1 | 324 | 1 | 49 | 1 |
| 1001114 | PD | 0 | 0.0 | 0.0 | 61.0 | Male | 30 | 2L | N | 195.0 | 6.4 | 1 | 112 | 1 | 112 | 1 |
| 1001117 | PD | 1 | 0.0 | 0.0 | 73.0 | Male | 72 | 1L | N | 201.7 | NA | NA | 23 | 1 | 23 | 1 |
| 1002004 | PD | 1 | 1.0 | 2.0 | 46.0 | Male | 53 | 1L | N | 132.0 | 4.3 | 1 | 24 | 1 | 24 | 1 |
| 1002015 | PD | 1 | 4.0 | 0.4 | 66.0 | Male | 64 | 3L+ | N | 227.0 | 4.9 | 1 | 126 | 1 | 53 | 1 |
| 1002019 | SD | 1 | 0.0 | 3.0 | 60.0 | Female | 21 | 3L+ | Y | 119.0 | 4.4 | 0 | 349 | 1 | 56 | 1 |
| 1002023 | SD | 1 | 0.0 | 0.5 | 48.0 | Male | 61 | 2L | N | 139.0 | 15.5 | 1 | 231 | 1 | 167 | 1 |
| 1002024 | SD | 1 | 0.0 | 0.0 | 50.0 | Male | 73 | 1L | Y | 157.0 | 11.9 | 0 | 188 | 1 | 56 | 0 |
| 1004011 | PR | 1 | 0.0 | 0.5 | 61.0 | Male | 175 | 3L+ | Y | 187.0 | NA | NA | 560 | 0 | 502 | 0 |
| 1004012 | PD | 1 | 0.0 | 0.0 | 61.0 | Male | 17 | 3L+ | N | 234.0 | 12.8 | 0 | 521 | 0 | 52 | 1 |
| 1004013 | PD | 1 | 0.0 | 2.0 | 59.0 | Male | 22 | 2L | Y | 140.0 | 3 | 1 | 159 | 0 | 54 | 1 |
| 1004014 | SD | 1 | 0.0 | 1.0 | 63.0 | Male | 36 | 2L | N | 209.0 | 18.1 | 1 | 497 | 0 | 171 | 1 |
| 1004018 | PD | 1 | 0.0 | 3.0 | 70.0 | Male | 47 | 2L | N | 169.0 | 4.3 | 0 | 89 | 1 | 57 | 1 |
| 1006005 | PD | 1 | 0.0 | 0.5 | 64.0 | Male | 132 | 2L | Y | 284.0 | 4.1 | 1 | 29 | 1 | 29 | 1 |
| 1007014 | uPR | 1 | 0.0 | 0.0 | 49.0 | Male | 77 | 3L+ | Y | 253.0 | 13.8 | 0 | 125 | 1 | 125 | 1 |
| 1007018 | SD | 1 | 0.0 | 0.0 | 49.0 | Male | 166 | 3L+ | Y | 335.0 | 3.6 | 1 | 209 | 1 | 113 | 1 |
| 1009017 | SD | 1 | 1.0 | 0.5 | 64.0 | Female | 138.8 | 2L | N | 208.0 | 4.5 | 0 | 96 | 1 | 96 | 1 |
| 1009018 | PD | 1 | 0.0 | 0.0 | 72.0 | Female | 193 | 1L | Y | 792.0 | 6.7 | 1 | 102 | 1 | 64 | 1 |
| 1009026 | PD | 1 | 0.0 | 1.0 | 61.0 | Male | 32 | 2L | Y | 136.0 | 13.1 | 1 | 142 | 1 | 1 | 0 |
| 1009027 | PD | 1 | 0.0 | 0.0 | 58.0 | Female | 73.7 | 3L+ | N | 237.0 | 7.7 | 1 | 27 | 0 | 1 | 0 |
| 1009029 | PD | 1 | 0.0 | 0.0 | 56.0 | Male | 38 | 3L+ | N | 152.0 | 2.7 | 0 | 134 | 1 | 1 | 0 |

| **Subject ID** | **Therapeutic effect** | **ECOG** | **%TC PD-L1** | **%IC PD-L1** | **Age** | **Sex** | **Tumor burden (mm)** | **Prior treatment** | **Liver metastasis** | **LDH (U/L)** | **TMB Muts/Mb** | **11q13 amplification** | **OS** | **State** | **PFS** | **State** |
|---|---|---|---|---|---|---|---|---|---|---|---|---|---|---|---|---|
| 1011007 | PD | 1 | 0.0 | 1.0 | 61.0 | Male | 173 | 1L | Y | 985.0 | NA | 1 | 39 | 1 | 39 | 1 |
| 1014001 | PD | 1 | 0.0 | 0.0 | 62.0 | Female | 91 | 3L+ | N | 487.0 | 3.4 | 1 | 527 | 0 | 56 | 1 |
| 1014011 | PD | 1 | 0.0 | 0.5 | 53.0 | Male | 72 | 2L | Y | 452.0 | 4.3 | 0 | 51 | 1 | 51 | 1 |
| 1014016 | PR | 1 | NA | NA | 65.0 | Male | 42 | 2L | N | 191.0 | 0 | 0 | 517 | 0 | 422 | 0 |
| 1015004 | PR | 1 | 20.0 | 10.0 | 68.0 | Female | 28 | 3L+ | N | 199.0 | 3 | 0 | 574 | 0 | 508 | 0 |
| 1015005 | PR | 1 | 0.0 | 0.5 | 55.0 | Male | 48 | 2L | N | 147.0 | 6.2 | 0 | 535 | 0 | 428 | 1 |
| 1015006 | SD | 1 | 0.0 | 0.0 | 64.0 | Male | 22 | 1L | N | 175.0 | NA | NA | 512 | 1 | 337 | 0 |
| 1015008 | PD | 1 | 0.0 | 0.0 | 53.0 | Male | 113.7 | 3L+ | N | 176.0 | NA | NA | 28 | 1 | 28 | 1 |
| 1015011 | SD | 1 | 2.0 | 0.5 | 64.0 | Male | 27.2 | 3L+ | N | 186.0 | 16.7 | 1 | 536 | 0 | 275 | 1 |
| 1015012 | PD | 1 | 0 | 0 | 55 | Female | 172.4 | 1L | N | 241 | 0.1 | 0 | 48 | 1 | 48 | 1 |
| 1016014 | SD | 1 | 70.0 | 2.0 | 71.0 | Male | 22 | 3L+ | N | 149.0 | 7.1 | 0 | 203 | 1 | 203 | 1 |
| 1016016 | SD | 1 | 0.0 | 0.0 | 64.0 | Male | 10 | 3L+ | N | 112.0 | NA | 0 | 532 | 0 | 342 | 0 |
| 1016017 | PR | 1 | 0.0 | 0.5 | 62.0 | Male | 44 | 3L+ | N | 430.0 | 1.4 | 0 | 581 | 0 | 507 | 0 |
| 1016021 | PD | 1 | 0.0 | 0.0 | 57.0 | Male | 45 | 3L+ | N | 129.0 | 4 | 0 | 143 | 1 | 54 | 1 |
| 1016023 | SD | 1 | 0.0 | 0.5 | 49.0 | Male | 62 | 3L+ | N | 227.0 | 5.7 | 1 | 72 | 1 | 72 | 1 |
| 1016024 | PD | 1 | 0.0 | 0.0 | 67.0 | Male | 23 | 3L+ | N | 184.0 | 12 | 1 | 350 | 1 | 58 | 1 |
| 1016027 | SD | 1 | 10.0 | 0.5 | 56.0 | Male | 21 | 1L | N | 100.0 | 3 | 1 | 169 | 1 | 122 | 1 |
| 1016029 | PD | 1 | 0.0 | 0.0 | 52.0 | Male | 102 | 1L | N | 153.0 | 3.9 | 0 | 356 | 1 | 56 | 1 |
| 1016030 | SD | 1 | 0.0 | 15.0 | 58.0 | Male | 68 | 1L | N | 184.0 | NA | 1 | 149 | 1 | 149 | 1 |
| 1017004 | PD | 1 | 0.0 | 0.0 | 53.0 | Male | 59.5 | 3L+ | N | 114.0 | 5.2 | 1 | 170 | 1 | 55 | 1 |
| 1018001 | PR | 1 | 0.0 | 0.0 | 68.0 | Male | 124 | 3L+ | N | 252.0 | NA | NA | 528 | 0 | 168 | 1 |
| 1019006 | PD | 1 | 0.0 | 0.0 | 68.0 | Male | 14 | 3L+ | N | 255.0 | NA | NA | 115 | 1 | 57 | 1 |
| 1019007 | PD | 1 | 0.0 | 0.0 | 50.0 | Male | 21 | 2L | N | 139.0 | NA | NA | 210 | 1 | 60 | 1 |
| 1019012 | PD | 1 | NA | NA | 58.0 | Male | 84 | 3L+ | N | 243.0 | NA | NA | 194 | 1 | 56 | 1 |
| 1020010 | PD | 1 | 2.0 | 1.0 | 60.0 | Male | 87 | 3L+ | Y | 177.0 | 8.8 | 1 | 179 | 1 | 56 | 1 |
| 1021003 | PR | 1 | 0.0 | 0.5 | 62.0 | Male | 37.5 | 3L+ | N | 145.0 | 8.4 | 1 | 543 | 0 | 347 | 1 |
| 1021004 | SD | 1 | 0.0 | 0.5 | 60.0 | Male | 105.5 | 2L | N | 165.0 | 1.5 | 0 | 513 | 0 | 87 | 0 |
| 1022005 | PD | 1 | 0.0 | 0.5 | 57.0 | Male | 50.2 | 2L | Y | 209.0 | 2.6 | 1 | 97 | 1 | 57 | 1 |
| 1023022 | PD | 1 | 0.0 | 0.0 | 64.0 | Male | 154 | 1L | Y | 794.0 | 16.5 | 0 | 34 | 1 | 34 | 1 |
| 1023025 | SD | 1 | 0.0 | 0.5 | 54.0 | Male | 74 | 3L+ | N | 283.0 | 12.4 | 0 | 443 | 1 | 112 | 1 |
| 1023026 | SD | 1 | 0.0 | 3.0 | 66.0 | Male | 21 | 2L | N | 247.0 | 6.4 | 0 | 420 | 1 | 280 | 1 |
| 1024010 | PD | 0 | 0.0 | 0.0 | 67.0 | Male | 106 | 2L | Y | 204.7 | 11.9 | 0 | 49 | 1 | 19 | 1 |

| **Subject ID** | **Therapeutic effect** | **ECOG** | **% TC PD-L1** | **%IC PD-L1** | **Age** | **Sex** | **Tumor burden (mm)** | **Prior treatment** | **Liver metastasis** | **LDH (U/L)** | **TMB Muts/Mb** | **11q13 amplification** | **OS** | **State** | **PFS** | **State** |
|---|---|---|---|---|---|---|---|---|---|---|---|---|---|---|---|---|
| 1024012 | CR | 0 | 0.0 | 0.0 | 59.0 | Male | 31.7 | 1L | N | 260.3 | 6 | 0 | 501 | 0 | 337 | 0 |
| 1024015 | PR | 0 | 0.0 | 5.0 | 54.0 | Male | 48.4 | 1L | N | 175.0 | 12.9 | 0 | 506 | 0 | 506 | 0 |
| 1024016 | SD | 1 | 0 | 0.5 | 73 | Male | 24.4 | 3L+ | N | 116.6 | 18.6 | 1 | 253 | 1 | 119 | 1 |

### Example 4. Mutation profile for anti-PD-1 treatment and its corresponding relation with therapeutic effect

### 4.1 Whole exome sequencing (WES) and data analysis

Tumor tissues and matched PBMC samples were collected, 4 µm sections of hematoxylin and eosin-stained FFPE samples were pathologically examined to ensure that each sample had a nucleated cytology of greater than 80% and a tumor content of no less than 20%. No less than 200 ng of DNA was extracted using enough unstained FFPE sections. About 200 µL of PBMCs matched to the general generation of 1-5 µg of DNA were used as reference.

Library preparation was performed by the ultrasonic fragmentation of double stranded DNA to 250 bp. The library was constructed by end repair, dA addition, and adapter ligation using the SureSelect XT library Prep kit or the KAPA Hyper Prep kit (KAPA biosystems), and then PCR amplification and quantification by Qubit evaluation were performed. Target regions were captured by hybridization according to protocol and libraries were prepared. Sequence reads were generated using Illumina NovaSeq 6000 (Illumina Inc., San Diego, CA). The data quality was checked and controlled and a set of customized bioinformatics pipelines was established for finding SNVs, long and short insertions and deletions, CNAs, gene rearrangements, TMBs and MSIs. Finally, all mutations detected were treated clinically against our internal annotation database. According to the annotated mutations, a concise report based on clinical trials and literature will be generated.

### 4.2 Identification of somatic signal-to-noise ratio, short indentation and copy number alterations (SCNAs)

The original reads were aligned to the human genome reference sequence (hg19) using a Burrows-Wheeler sequencer and PCR repeats were removed using the Picard marker replication algorithm. The original calls for SNV and S-indel were made by an internal algorithm. At least 5 reads were required to support the optional call. Variations with read depths less than 50×, chain bias less or greater than 90%, VAF < 1% were removed. SNPs are generally defined as frequencies greater than 1.5% from the dbSNP database (version 147) or from the Exome sequencing project 6500 (ESP6500) or frequencies greater than 1.5% from the 1000 Genomes Project which are excluded for further consideration. Products from sequencing errors or populations are also excluded.

To define SCNA events, alignment reads within each exon region were normalized, and the results were further corrected for GC content and reference genome mappability using CNVKIT (version 0.9.1). The obtained replication rate was partitioned by a cyclic binary partitioning algorithm. The proportion of aberrated tumor cells was estimated with the allele frequency of over 4000 sequenced SNPs (single nucleotide polymorphisms) according to ASCAT. The copy rate of the tumor tissue and the matched normal blood sample were calculated through depth normalization, GC and mappability correction. The fragment was considered to be enlarged or deleted if log2 (copy rate) was more than 0.8 or no more than -1.

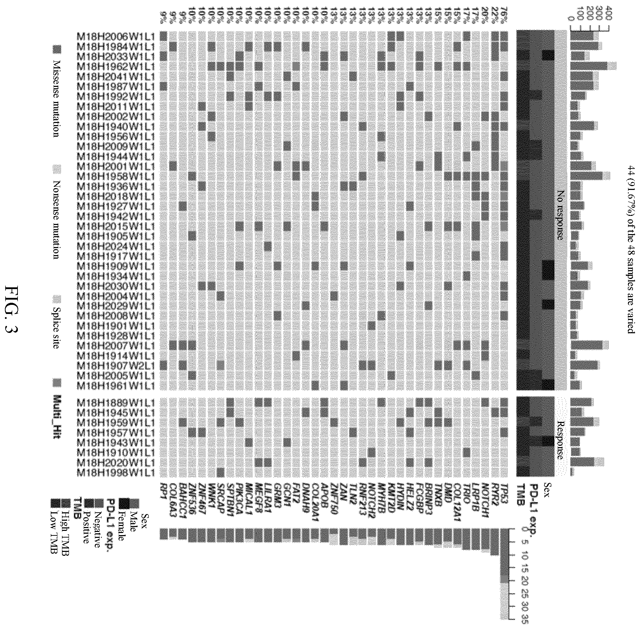

Genomic map of WES showed that missense mutations or truncations of TP53 (p53) (76%), RYR2 (22%), NOTCH1 (20%), LRP1B (17%) and TRIO (17%) were the top 5 mutations in ESCC tumor biopsies in this study (FIG. 3). However, missense mutations or truncations of those five genes or loss of other functional mutations was not associated with clinical response.

### Example 5. Relation between 11q13 gene amplification and resistance to anti-PD-1 treatment

Whole transcriptome sequencing (RNA sequence) was performed by the following method. RNA was extracted from unstained FFPE sections using the miRNeasy kit (Cat No: 217504, Qiagen), and further treated to delete rRNA using the NEBNext^{®} rRNA depletion kit (Cat No: E6310L, New England Biolabs). First and second strand cDNAs were synthesized using the M-MLV RT RNase (H-) (Cat No: M3683, Promega) and NEB second strand mRNA synthesis kit (Cat No: E6111L, New England Biolabs), respectively. The cDNA product was sonicated to produce a fragment of about 200 bp (Covaris E220). The adapter-ligated library was constructed from cDNAs using KAPA Hyper Prep kit (Cat. No: 07962363001, Roche), and sequenced on the NovaSeq 5000/6000 platform. The relative abundance of each annotated transcript was expressed in transcripts per million (TPM) and log2 transformation prior to analysis.

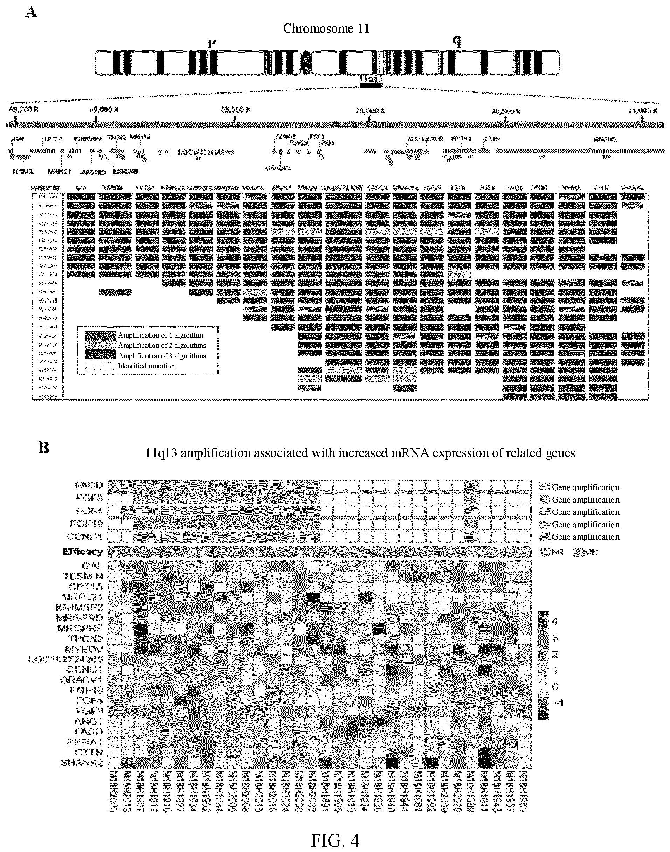

WES analysis of Example 4 showed that 24 (48.0%) of the 50 biopsies contained the amplification of the chromosome 11q13 region (FIG. 4A). The analysis of messenger RNA expression further confirmed genomic DNA amplification events and mRNA levels correlated with the amplification status of the corresponding genes in this region, including CCND1(Cyclin D1) and FGF family members (FGF3/4/19) (FIG. 4B). Importantly, patients without 11q13 amplification (n = 26) had statistically better objective response rates (30.8% vs. 4.2%, *p* = 0.024) (FIG. 5A) and longer PFS (3.7 months vs. 1.0 month; HR = 0.47 [95% CI 0.24 to 0.91], p = 0.025) than patients with 11q13 amplification (n = 24). Patients without 11q13 amplification also had longer median OS (11.5 months vs. 5.6 months; HR = 0.60 [95% CI 0.30 to 1.20]) (FIG. 5C).

### Example 6. Other biomarkers and subgroup analysis

Other biomarkers or subgroup analysis related to clinical effects includes: age, sex, ECOG score, prior treatment, presence of liver metastases at baseline, tumor volume, and baseline serum LDH levels. Among these, patients with an ECOG score of 0, low baseline tumor volume and baseline LDH levels above the upper limit of normal have numerically better ORR However, none of these differences was statistically significant (Table 4).

**Table 4: Correlation of other markers and subgroups with clinical effects**

| Characteristics | Numerical value | Number of people | Optimal response (%) | 95% CI (%) |
|---|---|---|---|---|
| Age | ≤60 | 30 | 16.7 | 5.6-34.7 |
| | >60 | 29 | 20.7 | 8.0-39.7 |
| Sex | Male | 52 | 19.2 | 9.6-32.5 |
| | Female | 7 | 14.3 | 0.4-57.9 |
| ECOG | 0 | 5 | 40.0 | 5.3-85.3 |
| | 1 | 54 | 16.7 | 7.9-29.3 |
| Prior treatment | 1 | 13 | 15.4 | 1.9-45.4 |
| | 2 | 19 | 15.8 | 3.4-39.6 |
| | 3+ | 27 | 22.2 | 8.6-42.3 |
| Liver metastasis | Yes | 16 | 18.8 | 4.0-45.6 |
| | No | 43 | 18.6 | 8.4-33.4 |
| Tumor volume | ≤100 mm | 46 | 19.6 | 9.4-33.9 |
| Baseline target lesion | >100 mm | 13 | 15.4 | 1.9-45.4 |
| LDH | Normal | 45 | 15.6 | 6.5-29.5 |
| | >ULN | 14 | 28.6 | 8.4-58.1 |
| Total | | 59 | 18.6 | 9.7-30.9 |

### Example 7. Study on inhibitory effect of anti-PD-1 antibody and CDK4/6 inhibitor on tumor growth in mice

CT-26 cells were seeded subcutaneously on the right side of hPD-1 humanized female mice at a concentration of 2.5 × 10⁵ cells/0.1 mL, and the mice were randomly grouped by tumor volume when tumors grew to about 93 mm³ with 7 mice per group, for a total of 4 groups:
G1: Anti-KLH hIgG4 (1 mg/kg) negative control group;
G2: toripalimab (Toripalimab) (1 mg/kg) group;
G3: palbociclib (100 mg/kg);
G4: toripalimab (1 mg/kg) in combination with palbociclib (100 mg/kg).

Anti-KLH hIgG4 and the toripalimab were administered by intraperitoneal injection, twice a week for three consecutive administrations, and palbociclib was administered orally, once a day. The experiment was ended 11 days after the first administration. Tumor volume and body weight of mice were measured and recorded twice a week. At the end of the experiment, animals were euthanized and tumor growth inhibition (TGI_{Tv}%) was calculated.

The results are shown in Table 5 and FIG. 7. The test drug palbociclib has a certain inhibitory effect on the tumor growth; compared with the administration of toripalimab (1 mg/kg) and palbociclib (100 mg/kg) alone, the administration of toripalimab (1 mg/kg) in combination with palbociclib (100 mg/kg) has more remarkable inhibitory effect on the tumor growth.

**Table 5. Effect of toripalimab in combination with RT391 on tumor volume of CT-26 cell-transplanted B-hPD-1 mice**

| Group | Test drug | Tumor volume (mm³)^{a} | | | *P*^{b} |
|---|---|---|---|---|---|
| | | Before administration | 11 days after the first administration | TGI (%) | |
| G1 | Anti KLH hIgG4 | 93±12 | 2731±217 | - | - |
| G2 | toripalimab (1 mg/kg) | 92±10 | 2519±320 | 8.0 | 0.592 |
| G3 | palbociclib (100 mg/kg) | 93±11 | 1550±189 | 44.8 | 0.001 |
| G4 | toripalimab + palbociclib (1 + 100 mg/kg) | 93±13 | 755±56 | 74.9 | <0.001 |

| | | | | | |
|---|---|---|---|---|---|
| Note: a: mean ± standard error; b: the statistical comparison of the tumor volume of the administration groups with tumor volumes of the Anti-KLH hIgG4 control group at 11 days after the administration, t-test. | | | | | |

## Claims

1. Use of an anti-PD-1 antibody and/or an antigen-binding fragment thereof alone or in combination with an additional anti-cancer agent other than the anti-PD-1 antibody, in the preparation of a medicament for treating a solid tumor, wherein the solid tumor does not have a gene amplification of the chromosome 11q13 region.

2. The use according to claim 1, wherein the anti-cancer agent is a small molecule targeted anti-cancer agent.

3. The use according to claim 1, wherein the anti-cancer agent is selected from one or more of a CDK4/6 inhibitor and an FGF/FGFR inhibitor; preferably, the CDK4/6 inhibitor is selected from ribociclib and palbociclib.

4. Use of one or more of a CDK4/6 inhibitor and an FGF/FGFR inhibitor alone or in combination with an anti-PD-1 antibody and/or an antigen-binding fragment thereof, in the preparation of a medicament for treating a solid tumor, wherein the solid tumor has a gene amplification of the chromosome 11q13 region; preferably, the CDK4/6 inhibitor is selected from ribociclib and palbociclib.

5. The use according to any one of claims 1 to 4, wherein the anti-PD-1 antibody is a monoclonal antibody or an antigen-binding fragment thereof specifically binding to PD-1 and blocking the binding of PD-L1 to PD-1.

6. The use according to claim 5, wherein in the anti-PD-1 antibody, the light chain complementarity determining region comprises amino acid sequences set forth in SEQ ID NOs: 1, 2 and 3, and the heavy chain complementarity determining region comprises amino acid sequences set forth in SEQ ID NOs: 4, 5 and 6.

7. The use according to claim 5, wherein in the anti-PD-1 antibody, the light chain variable region comprises an amino acid sequence set forth in SEQ ID NO: 7, and the heavy chain variable region comprises an amino acid sequence set forth in SEQ ID NO: 8.

8. The use according to claim 5, wherein in the anti-PD-1 antibody, the light chain comprises an amino acid sequence set forth in SEQ ID NO: 9, and the heavy chain comprises an amino acid sequence set forth in SEQ ID NO: 10.

9. The use according to claim 5, wherein the anti-PD-1 antibody is selected from nivolumab, pamtuzumab, toripalimab, sintilimab, camrelizumab and tislelizumab, or a combination thereof.

10. The use according to any one of claims 1 to 9, wherein the anti-PD-1 antibody or the antigen-binding fragment thereof is administered at a dose of about 0.1 mg/kg to about 10.0 mg/kg body weight, e.g., about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight, or selected from a fixed dose of about 120 mg to about 480 mg, e.g., a fixed dose of 120 mg, 240 mg, 360 mg or 480 mg.

11. The use according to claim 10, wherein the anti-PD-1 antibody or the antigen-binding fragment thereof is administered at a frequency of about once every week, once every two weeks, once every three weeks, once every four weeks or once a month, preferably once every two weeks.

12. The use according to claim 10, wherein the anti-PD-1 antibody or the antigen-binding fragment thereof is administered at a dose of 1 mg/kg body weight, 3 mg/kg body weight or 10 mg/kg body weight, or of a fixed dose of 240 mg or 480 mg once every two weeks.

13. The use according to claim 10, wherein the anti-PD-1 antibody or the antigen-binding fragment thereof is administered parenterally, e.g., by intravenous infusion, in a liquid dosage form, e.g., an injection.

14. The use according to claim 10, wherein the anti-PD-1 antibody or the antigen-binding fragment thereof is administered for a period of one week, two weeks, three weeks, one month, two months, three months, four months, five months, half a year or longer, optionally, the duration of each administration period is the same or different, and the interval between each administration period is the same or different.

15. A method for treating a solid tumor, comprising sequencing an individual prior to treatment, wherein an individual not having an amplification of the chromosome 11q13 region is administered with an anti-PD-1 antibody or an antigen-binding fragment thereof alone, optionally in combination with one of or a combination of more of a CDK4/6 inhibitor and/or an FGF/FGFR inhibitor; an individual having an amplification of the chromosome 11q13 region is administered with one of or a combination of more of a CDK4/6 inhibitor and/or an FGF/FGFR inhibitor alone, optionally in combination with an anti-PD-1 antibody or an antigen-binding fragment thereof; preferably, the CDK4/6 inhibitor is selected from ribociclib and palbociclib.

16. The use according to any one of claims 1 to 14 or the method according to claim 15, wherein the solid tumor is esophageal cancer or colon cancer.

17. The use or method according to claim 16, wherein the esophageal cancer is esophageal squamous cell carcinoma.

18. Use of a reagent for detecting a gene amplification of the chromosome 11q13 region in the preparation of a test kit for predicting the therapeutic effect of the anti-PD-1 antibody and/or the antigen-binding fragment thereof on a tumor patient.

19. Use of a reagent for detecting a CDK4/6 gene amplification in the preparation of a test kit for predicting the therapeutic effect of the anti-PD-1 antibody and/or the antigen-binding fragment thereof on a tumor patient.

20. Use of a reagent for detecting an FGF/FGFR gene amplification in the preparation of a test kit for predicting the therapeutic effect of the anti-PD-1 antibody and/or the antigen-binding fragment thereof on a tumor patient.

21. A kit for treating an individual with cancer, comprising: (a) an anti-PD-1 antibody or an antigen-binding fragment thereof, and optionally an additional anti-cancer agent other than the anti-PD-1 antibody or the antigen-binding fragment thereof; and (b) instructions for administering the anti-PD-1 antibody or the antigen-binding fragment thereof alone, or in combination with an additional anti-cancer agent other than the anti-PD-1 antibody or the antigen-binding fragment thereof, to treat cancer in the individual; the cancer is a solid tumor; as a preferred embodiment, the cancer includes, but is not limited to, gastric cancer, esophageal cancer, nasopharyngeal cancer, head and neck squamous cell carcinoma, breast cancer, bladder cancer, and colon cancer; preferably, the additional anti-cancer agent is a CDK4/6 inhibitor, more preferably ribociclib and palbociclib.
